# EUROPEAN PATENT APPLICATION

(11) **EP 4 285 915 A1**
(43) Date of publication of application: **06.12.2023**
(21) Application number: 22177263.5
(22) Date of filing: 03.06.2022
(51) Int. Cl.: A61K 35/28, A61P 9/00, A61P 11/00, C12N 5/0775

(54) **HUMAN MESENCHYMAL STEM CELL-CONDITIONED MEDIUM FOR USE IN TREATMENT OF CHRONIC HEART-LUNG AND VASCULAR DISEASES, IN PARTICULAR, OF PULMONARY ARTERIAL HYPERTENSION (PAH)**

(71) Applicant: Medizinische Hochschule Hannover, 30625 Hannover (DE); Academisch Ziekenhuis Leiden h.o.d.n. LUMC, 2333 ZA Leiden (NL)
(72) Inventor: Hansmann, Georg, 30171 Hannover (DE); Hass, Ralf, 30655 Hannover (DE); Giera, Martin, 1013 XL Amsterdam (NL); Ralser, Markus, 13505 Berlin (DE)
(74) Representative: Moré, Solveig Helga

(57) **Abstract**

The present invention relates to the field of medical treatment of chronic disease with stem-cell derived products, in particular, human mesenchymal stem cell-derived treatment of chronic heart-lung and vascular diseases. The inventors provide human mesenchymal stem cell-conditioned medium, such as human umbilical cord mesenchymal stem cell-conditioned medium (HUMSC-CM) for use in treatment of chronic heart-lung and vascular diseases, in particular, pulmonary hypertension (PH, groups 1-5), such as pulmonary arterial hypertension (PAH; group 1 PH according to World Symposium on Pulmonary Hypertension 2018, http://www.wsphassociation.org/). The invention also provides a method of treating chronic heart-lung-vascular diseases by means of administering such conditioned medium intravascularily.

## Description

The present invention relates to the field of medical treatment of chronic disease with stem-cell derived products, in particular, human mesenchymal stem cell-derived treatment of chronic heart-lung and vascular diseases. The inventors provide human mesenchymal stem cell-conditioned medium, such as human umbilical cord mesenchymal stem cell-conditioned medium (HUMSC-CM) for use in treatment of chronic heart-lung and vascular diseases, in particular, pulmonary hypertension (PH, groups 1-5), such as pulmonary arterial hypertension (PAH; group 1 PH according to World Symposium on Pulmonary Hypertension 2018, http://www.wsphassociation.org/). The invention also provides a method of treating chronic heart-lung and vascular diseases by means of administering such conditioned medium intravascularily.

Chronic heart-lung and vascular diseases are debilitating conditions that affect the right and/or left heart (ventricles, atria), the interconnecting vessels between heart and lung, in particular the peripheral, normally non-muscularized pulmonary arteries, but also the smaller coronary arteries supplying the heart muscle, and often also the pulmonary parenchyma and interstitium (in particular, in group 3 PH) and lymphatic vessels (in particular, in group 2 PH, combined pre and postcapillary PH, and PH associated with complex congenital heart disease).

Chronic heart-lung and vascular diseases comprise different disease complexes, genetic syndromes and conditions, e.g., PH (precapillary, postcapillary, or a combination of the two), in particular, PAH, pulmonary fibrosis, chronic obstructive pulmonary disease, bronchopulmonary dysplasia, and other chronic progressive lung diseases, and chronic heart diseases.

All, pulmonary arteries, pulmonary arterioles, capillaries, lymphatic vessels, pulomonary venuoles and pulmonary veins can be affected in group 1 PH (PAH), and other forms of PH (groups 2-5). The heart and lungs are intricately related; whenever the heart is affected by a disease, the lungs risk following and vice versa.

To be classified as pulmonary heart disease, the cause must originate in the pulmonary circulation system; right ventriclular hypertrophydue to a systemic defect such as amyloidosis is not classified as pulmonary heart disease. Two exemplary causes are vascular changes as a result of tissue damage (e.g. disease, hypoxic injury, genetic mutations, idiopathic), and chronic hypoxic pulmonary vasoconstriction, for example, in high altitude. If left untreated, progressive heart-lung-vascular disease such as PAH is lethal.

PAH, a particularly progressive, chronic heart-lung-vascular disease, is characterized by obliteration and/or loss of peripheral pulmonary arteries, a consecutive increase in pulmonary vascular resistance, and consecutive high arterial pressure in the lungs (pulmonary hypertension), resulting in hypertrophy, dilation and ultimately failure of the right ventricle (RV) of the heart. Cardiac hypertrophy is an adaptive response to a long-term increase in pressure afterload (pulmonary artery pressure for the RV). Individual cardiac muscle cells enlarge in size, undergo metabolic and structural changes, to drive the increased contractile force required to move the blood against greater resistance.

The pathobiology of pulmonary vascular disease (PVD) and PAH is complex, multifactorial, and driven by inflammation and metabolic dysfunction. Despite remarkable improvements in pharmacotherapy (Galie et al. 2019, Hansmann et al. 2019), advanced PAH is still a non-curable, debilitating and fatal condition (Hansmann, 2017). Many patients (20-30%) progress to lung transplant or death within 5 years after diagnoses, with certain subgroups having the highest risk (e.g., heritable PAH with certain mutations, complex congenital heart disease, connective tissue disease).

Today, PAH is considered a systemic disease that is strongly associated with pathologiocal involvement of multiple organs and processes in and outside the chest, including the eyes, kidneys, liver, adipose tissue and skeletal muscle (Nickel et al., 2020; Hamberger et al., 2022).

Moreover, PAH and RV failure have systemic consequences on multiple organ systems, driving self-perpetuating pathophysiological mechanisms, aspects of increased susceptibility of organ damage, with reciprocal impact on the course of the disease (Rosenkranz et al., 2020).

Transforming growth factor beta (TGFβ) receptor superfamily members (bone morphogenetic protein receptor 2, BMPR2; activin A receptor like type 1, ACVRL1; endoglin, ENG) and their ligands play a critical role in the etiology of PAH (Trembath et al., 2001; Calvier et al., 2017; Humbert et al., 2019; Morell et al., 2019). Heterozygous, loss-of-function mutations in the BMPR2, ACVRL1 and ENG genes, among others, have been described in familial/heritable PAH (HPAH) and idiopathic PAH (IPAH); such mutations are also found in hereditary hemorrhagic telangiectasia (HHT; Osler-Weber-Rendu disease). Patients with ACVRL1 mutations who do develop PAH (Trembath et al., 2001) are particularly young, have often rapid disease progression and a prognosis that is worse than for those with BMPR2 mutations (Girerd et al., 2010).

The inventors previously demonstrated in the SuHx rat model of PAH (VEGFR2 blockade plus 3 weeks hypoxia) that the PPAPγ agonist pioglitazone fully reverses severe PAH, pulmonary vascular remodeling and vessel loss, and prevents RV failure by boosting fatty acid oxidation (FAO) and decreasing intracardiac lipid accumulation (Legchenko et al., 2018). PPAPγ activation by pioglitazone is one of the very few interventions that fully reverses both PAH and pulmonary vessel loss, and induces angiogenesis genes and capillaries in the hypertensive RV of the SuHx rat ( Legchenko et al., 2018; Zelt et al., 2019).

Stem and progenitor cells and/or their secreted products may also represent efficient therapies for PAH (Granton et al., 2015; Klinke et al., 2020; Hansmann et al., 2012). The endogenous role for mesenchymal stem cells (MSCs) is maintenance of stem cell niches (classically the hematopoietic), and as such, MSCs participate in organ homeostasis, wound healing, and successful aging. Mesenchymal stem cells have been proposed as extremely promising therapeutic agent for heart-lung tissue regeneration. However, long-term engraftment of MSC has never been demonstrated in preclinical or clinical studies, and the MSCs' major beneficial effects are proposed to be of paracrine nature, i.e., mediated by secreted factors. Neonatal MSCs such as human umbilcal cord-derives MSC (HUCMSCs or HUC-MSCs) are thought to have even more powerful regenerative capacity than the classical "adult" MSCs derived from adult human tissues, e.g., bone marrow, adipose tissue, peripheral blood.

Human MSCs are thought to be immunologically inert, as are cell-free HUCMSC-CM-infusions. However, long-term tissue engraftment of MSCs has never been demonstrated in preclinical or clinical studies.

MSC-derived extracellular vesicles (EVs) (Shah et al., 2018), isolated from CM, have marked efficiency in hyperoxia-induced newborn bronchopulmonary dysplasia in mice (Willis et al., 2018) and in *VEGFR2-*blocked/hypoxia-exposed rats with PAH and RV failure (Klinger et al., 2020). In the latter study, *repetitive* dosing of bone-marrow-derived MSC-EVs within days was most efficient in reversing PAH (Klinger et al., 2020). Yet, the preclinically used EVs are frequently poorly defined (Mitsialis, 2020), making inter-study comparisons and GMP-certified therapies for clinical use a difficult task.

In light of this, the inventors addressed the problem of providing a treatment for chronic heart-lung and vascular disease that is effective in human subjects, and, which may advantageously overcome one or more disadvantages of previously known treatment.

This problem is solved by the invention, in particular, by the claimed subject matter.

The invention provides a human mesenchymal stem cell-conditioned medium for use in treatment of a chronic heart-lung and vascular disease in a human subject.

The inventors have for the first time shown that application of conditioned medium derived from human mesenchymal stem cells (MSCs) is a safe and effective treatment for chronic heart-lung and vascular disease such as for pulmonary arterial hypertension (PAH) in humans. A 3-year-old female presented with heritable PAH associated with hereditary hemorrhagic telangiectasia (HHT) and an underlying ACVRL1 gene mutation; she was treated for six months with serial intravascular infusions of conditioned media (CM) from allogenic human umbilical cord MSC (= HUCMSCs). The treatment markedly improved clinical and hemodynamic parameters, and decreased blood plasma markers of vascular fibrosis, injury and inflammation. A comparative analysis of single-cell RNA-seq data collected from three HUCMSC and two HUVEC control cell cultures identified eight common cell clusters, all of which indicated regenerative potential specific for HUCMSCs. The properties of HUCMSCs were validated by untargeted label-free quantitation of the cell and CM proteome suggesting increased activity of regeneration, autophagy, and anti-inflammation pathways, and mitochondrial function. Prostaglandin analysis demonstrated increased HUCMSC secretion of prostaglandin E₂, known for its regenerative capacity.

The chronic heart-lung and vascular disease treated in the context of the invention may be, e.g., pulmonary hypertension, pulmonary fibrosis, chronic obstructive pulmonary disease, bronchopulmonary dysplasia, other chronic progressive lung diseases (interstitial, parenchymal, developmental), chronic heart disease (including right and/or left heart failure), stroke, and/or peripheral arterial obliterative disease.

Treatment of heart-lung disease, in particular, pulmonary hypertension is a preferred embodiment of the invention. Pulmonary hypertension is classified into pulmonary hypertension groups 1-5 (Simmonneau et al., 2019). This includes pulmonary arterial hypertension (PAH, group 1) but also other forms of pulmonary hypertension (PH); e.g. PH associated with left heart disease (group 2 PH) or lung diseases (group 3 PH). Pulmonary fibrosis can be idiopathic (IPF) or acquired. Pulmonary fibrosis can be associated with PH, or it may occur without PH. Similarly, chronic obstructive pulmonary disease (COPD) can be treated in the absence or presence of PH. Other chronic progressive lung diseases that may be treated with the conditioned medium of the invention include interstitial and/or parenchymal lung diseases, such as cystic fibrosis and bronchiectasis. Bronchopulmonary dysplasia is a common developmental lung disease that can also be treated. It is also possible to treat chronic heart disease, specifically,
- left heart failure with (group 2 PH) or without increased left atrial pressure
- heart failure or right ventricular dysfunction in the setting of high RV pressure load
- heart failure with right and/or left ventricular dysfunction, due to coronary artery disease.

The conditioned medium of the invention has been shown to be particularly effective for treatment of pulmonary hypertension, preferably pulmonary arterial hypertension (PAH). Surprisingly, the inventors found that the present stem cell-based therapy may have superior efficiency vs. vasodilatory drugs, particularly in very aggressive, severe forms of preclinical or clinical PAH. The conditioned medium of the invention may thus advantageously be used for treatment of all forms of PAH (specifically, moderate to severe). Such forms are often found, e.g., along with mutations in PAH candidate genes (e.g., *ACVRL1, BMPR2),* and in proinflammatory conditions such as systemic sclerosis (connective tissue disease) (Hansmann et al., 2019; Galie et al., 2016).

Mutations in PAH candidate genes can be germline mutations or spontaneous mutations, usually resulting in loss-of-function of the protein. PAH candidate genes comprise *ACVRL1, BMPR2, ENG, CAV1, EDN1* and *SMAD* family genes, e.g. *SMAD4, SMAD9, AGTR1, BMPR1B, EDNRA, EIF2AK4, KCNA5, KCNK3, NOS2, NOTCH3, SERPINE1, SIRT3, SOX17, TBX4, THBS1, TOPBP1* and TRPC6 genes. Mutations in the *ACVRL1* gene have been found to lead, typically, to a particularly severe disease. The inventors could show that, wit the conditioned medium of the invention, even the most severe form of PAH, that is, heritable PAH with *ACVRL1*-mutations (Morrell et al., 2019), can be treated with human umbilical cord derived MSC-conditioned medium.

Primary vascular diseases other than PAH that can be treated with the invention include intra- and extracranial vascular disease affecting the brain (prototype: stroke) and peripheral arterial occlusive disease (risk factors: smoking, diabetes), both of which are associated with atherosclerosis, metabolic dysfunction/insulin resistance and obesity. Intriguingly, the latter two conditions are also commonly found in PAH patients as co-morbiodities (Hansmann et al., 2007; Zamanian et al., 2009; Poms et al., 2013; Agrawal et al., 2020).

The MSCs from which the conditioned medium is derived can be any human MSCs. In a preferred embodiment, they can be neonatal or birth-associated MSC, such as umbilical cord MSCs, amnion membrane MSCs, or plazental MSCs. Preferably, throughout the invention, they are umbilical cord mesenchymal stem cells. In this context, umbilical cord MSCs comprise MSCs isolated from whole umbilical cord, from Wharton's jelly or from umbilical cord blood. Isolation from whole umbilical cord is preferred, e.g., as described below or in the literature. Other MSCs, e.g., derived from adult human tissue, e.g., bone marrow, adipose tissue or peripheral blood, can also be used. Methods of preparing those different kinds of MSC are known in the art (e.g. Hass et al., 2011; Yang et al., 2016; Hoffmann et al., 2017).

Alternatively, human MSC cell lines, such as the human MSC544 cell line described in Melzer et al., 2020, can be employed for conditioning the medium.

The MSC comply with the minimal criteria proposed by the International Society for Cellular Therapy in 2006 (Dominici et al., 2006), in particular, expression of CD73, CD90 and CD105 and absence of at least CD14, CD31, CD34 and CD45, as well as presence of detectable G1, S, and G2/M phases.

MSCs can be derived from the human umbilical cord (whole umbilical cord or Wharton's jelly) following delivery of an infant, e.g., a full term infant. The cells may be cultivated by explant culture in MSC growth medium.

For example, umbilical cord tissue may be washed with phosphate buffered saline (PBS, pH 7.4) to remove blood cells, optionally, cut into pieces (e.g., approx. 1.5cm³ large) and incubated in MSC growth medium (e.g., αMEM (Invitrogen GmbH) supplemented with 10-20%, e.g., 15% of human serum (HS), preferably, allogeneic human AB-serum, penicillin (e.g., 100 units/mL), streptomycin (e.g., 100 mg/mL), and L-glutamine (e.g., 2mM) at 37°C in a humidified atmosphere with 5% CO₂). The explant culture may be performed for 7-21 days, e.g., 10-18, 11-17, 12-16, 13-15 or 14 days. The outgrowth of an adherent enriched MSC population may be harvested, e.g., by accutase (Capricorn Scientific GmbH, Ebsdorfergrund, Germany) treatment. The cells may be centrifuged, resuspended in MSC culture medium (e.g., αMEM) supplemented as described above and cultured at 37°C in a humidified atmosphere with 5% CO₂. The cells are preferably seeded at a density of 3000-4000 cells/cm², e.g., at 3.500 cells/cm². Harvesting and subculture into corresponding passages may again be performed following treatment with accutase (Capricorn Scientific GmbH), e.g., at 37°C for 3 min.

Before using the cells for conditioning media, continuously proliferating MSCs (or a sample thereof) should be harvested and analyzed for cell cycle progression and cell surface marker expression by flow cytometry. To confirm the identity of MSC, detectable G1, S, and G2/M phases, the presence of CD73, CD90, and CD105 with concomitant absence of CD14, CD31, CD34, CD45, and HLA-DR should be confirmed by FACS analysis, according to the suggestion by the International Society for Cellular Therapy as one of the minimal criteria for MSC characterization (Dominici et al., 2006).

The mesenchymal stem cell-conditioned medium of the invention is preferably harvested from a culture of MSC (e.g., UC-MSC) that is sub-confluent, so that the cells are in continuous growth phase, i.e., the cells are not confluent when the new (preferably serum-free) medium that is later harvested is added to the cells, and, preferably, the cells are also not yet confluent when the medium is harvested. For example, when the medium is added, the cells can be at a confluency of 70-80 %, e.g., 75%. When the medium is harvested, they can be at a confluency of 75-85 %, e.g., 80%.

The conditioned medium is serum-free medium obtainable, e.g., harvested from a sub-confluent culture of mesenchymal stem cells after 12 to 60 hours of culture, optionally, after 24 to 48 hours of culture, such as 30-36 hours of culture. The hours of culture are counted after new medium is added when the cells are seeded for a new passage. Before culturing the MSC in serum-free medium the culture is washed, e.g., three times with GMP-compatible PBS, e.g. DPBS CTS^{™} (Gibco GmbH). Because of the intended use for application to a human, the medium is preferably GMP-compatible serum-free medium without antibiotics. It can, e.g., be CTS STEMPRO MSC SFM XENOFREE basal Medium (Life Technologies, ThermoFisher GmbH). Accordingly, preferably, the conditioned medium is GMP-compatible serum-free medium, e.g., CTS STEMPRO MSC SFM XENOFREE basal Medium, harvested from a sub-confluent culture of mesenchymal stem cells after 24 to 48 hours of culture, such as 30-36 hours of culture.

After harvesting, the conditioned medium is preferably centrifuged to eliminate cells or cell debris, e.g, at about 3000 to 3500 g for 5-15 min. In the context of the invention, "about" is to be understood as +/- 10%. The conditioned medium is preferably not ultracentrifuged. To prevent infection of the subject by administration, absence of mycobacterial and bacterial contamination should be confirmed. The medium can also be tested for viral contamination and/or the donor can be screened for the absence of certain infections and/or antibodies, e.g., to exclude infection with HIV and Hepatitis virus.

The conditioned medium can be cryopreserved, e.g., at -20°C or, preferably, -80°C. It can also be used fresh or after short (preferably, 2 days or less or one day or less) storage at 4°C.

In contrast to prior art, where the high molecular weight (i.e., > 10 kDa) components of the conditioned media for animal trials had typically been concentrated via filtration, e.g., over a filter with a 10kDa membrane, often by a factor of 10, the inventors found that, surprisingly, conditioned media that had not been concentrated by filtration (i.e., wherein the high molecular weight components had not been concentrated by filtration compared to lower molecular weight components) are at least equally effective. Thus, preferably, the conditioned medium has not been concentratred via filtration. This allows for much more efficient production of the conditioned medium that can be employed for therapy.

The conditioned medium of the invention comprises extracellular vesicles, which are derived from the MSC.

In other prior art documents, isolated extracellular vesicles (EVs) from MSC-conditioned medium have been used for treatment of chronic heart-lung and vascular diseases in animal models. This is not the case in the context of the present invention. The conditioned medium of the present invention does not essentially or purely consist of isolated extracellular vesicles. Rather, it comprises both extracellular vesicles and components that are not comprised in the extracellular vesicles, e.g., free PGE₂. Preferably, the extracellular vesicles are not enriched in the conditioned medium. For example, no ultracentrifugation is carried out to isolate or enrich extracellular vesicles.

The inventors have found out that it is not required to isolate the extracellular vesicles, and the conditioned media per se can be used as safe and highly effective therapeutics. Again, this makes the preparation of the therapeutic agent significantly less laborious. In a preferred aspect, the conditioned medium, after centrifugation to remove cells or cellular debris, as described herein, is not further substantially changed. It may however be combined with other medicaments, if desired, e.g., in the context of a combination treatment further described below.

Further, for safety reasons, before or upon administration, the conditioned medium may be filtered, e.g., with a mesh size of 200 µm.

The inventors have further analysed the conditioned medium used in the invention, and identified several important components. Firstly, the conditioned medium comprises PGE₂, preferably, in an amount of at least 100 ng/mg protein. As further explained herein, PGE₂ is considered a highly active component of the medium.

Optionally, the conditioned medium further comprises PGF_{2α}, preferably, in an amount of at most 18 ng/mg protein. In contrast, the medium typically does not comprise detectable amounts of PGD₂.

The mesenchymal stem cell-conditioned medium of the invention further comprises proteins that are believed to act synergistically with the prostaglandins. For example, preferably, the conditioned medium comprises LRP1, APOE, MAPK11, FGF16 and/or LRP8, e.g., at least LRP1, APOE, MAPK11, FGF16, optimally, all of these proteins.

The MSC-CM of the invention is for use in treatment of chronic heart-lung and vascular disease, e.g., PAH and other forms of PH. Treatment according to the invention comprises administering the conditioned medium of the invention to a human subject with chronic heart-lung and vascular disease. The conditioned medium can be administered in any manner that allows the active agents in the medium, e.g., PGE2 or the proteins to contact tissue affected by the disease. For example, the conditioned medium may be administered intravascularly, in particular, intravenuously (peripherally, centrally venous), and/or to at least one pulmonary artery of the subject, and/or intracoronaryly. Different locations of administration can also be combined, e.g., a first dose may be administered to a pulmonary artery, and optionally, one or more further doses can be administered intravenuously. Administration to a pulmonary artery has been shown to be very effective by the experiments described herein. For example, an amount of conditioned medium can be administered to the right pulmonary artery, and another amount to the left pulmonary artery, e.g., half a dose each.

Further, a plurality of administrations to a pulmonary artery can also be considered, e.g., two, three, four or five such administrations. Alternatively, the conditioned medium can also be administered i.v. only to simplify procedures for the subject. The experiments show that this administration is also effective. In particular, for heart diseases like heart failure with right and/or left ventricular dysfunction, due to coronary artery disease, intracoronary application of HUCMSC-CM may be considered.

In the context of the invention, the dosis of the mesenchymal stem cell-conditioned medium of the invention can, based on the information provided herein, be determined by the responsible clinician. The dose and the number of doses to be administered depend on the subject and the parameters of the disease, e.g., on weight, age, sex, type and severity of disease. For example, the treatment may comprise administering a dose of 10 to 2000 mL of the conditioned medium, preferably, 100 to 500 mL or 200-400 mL. The target single dose for HUCMSC-derived conditioned medium is 10-20 mL/kg body weight, but lower or higher volumes may be safe and effective. In the experiment detailed herein, five doses of 200 mL of conditioned medium each were administered to a three-year old child (12.6kg body weight, 96cm body height).

The inventors have found that the first administration had the largest beneficial effect, particularily on hemodynamics. Thus, a single administration may be sufficient. Alternatively, repeated administrations may be carried out. For example, a treatment may comprise administering one to ten doses (e.g., two to nine doses, three to eight doses, four to seven doses, or five to six doses). The interval between the doses may be at least one day, e.g., 1 day to 6 months, 2-31 days, 3-30 days, 4-28 days, 5-21 days, 6-14 days, 7-10 days, or 8-9 days. Intervals may also vary, e.g., after intrapulmonary administration, a longer interval, e.g., about 7 days to several months may be suitable, while, after intravenous administration, an interval of about a day may be chosen.

The inventors have shown that the health status of the treated subject has improved and remained stable for at least three years. If the heart-lung and vascular disease again progresses, e.g., to a degree that endangers and/or impedes the subject, repeated treatment, e.g., about every three or five years to every 10 or even every 20 or 30 years can be considered.

As the inventors have been able to treat a subject with a very severe and progressive form of PAH in a very effective manner, a stand-alone treatment is possible. The treatment can also be combined with other medications intended to address the underlying disease mechanisms, e.g., a PPAPγ agonist such as pioglitazone and/or a 15-prostaglandin dehydrogenase (15-PGDH) inhibitor capable of blocking PGE₂ degradation, e.g., SW033291 (Zhang et al., 2015). In combination, e.g., with a 15-PGDH inhibitor, the dosis of conditioned medium may be reduced. Combination treatment may be administered simultaneously, together or separately, or at different time points within the same time period of, e.g., a month, a week, two days or a day. Such combination treatment may further comprise, in addition to the inventive treatment, administration of a PPAPγ agonist, a 15-PGDH inhibitor, and/or other so-called PAH-targeted medications such as phosphodiesterase 5 inhibitors, soluble guanylate cyclase stimulators, endothelin receptor antagonists, IP receptor agonists, prostacyclin analogues, or sotatercept, among other substances and medications.

The invention also provides a method of treating a chronic heart-lung and vascular disease in a human subject, e.g., PAH, comprising administering human mesenchymal stem cell-conditioned medium, e.g., HUMSC-CM as described herein, to said subject. The medium is administered intravascularily. Advantageously, an effective amount of the medium is admimistered. Further provided is the use of human mesenchymal stem cell-conditioned medium, e.g., HUMSC-CM as described herein for preparation of a pharmaceutical composition for treatment of a chronic heart-lung and vascular disease.

In the following, the invention is illustrated by reference to specific examples and embodiments. These are not intended to limit the invention.

Literature cited herein is herewith fully incorporated by reference.

### Legends

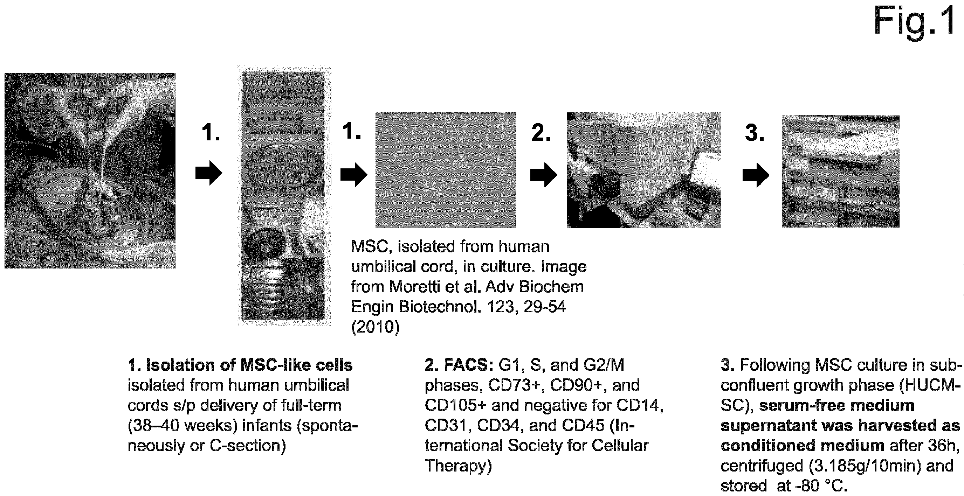

**Fig. 1** **Isolation of human umbilical cord mesenchymal stem cells (HUCMSC) and HUCMSC-conditioned media (CM)**

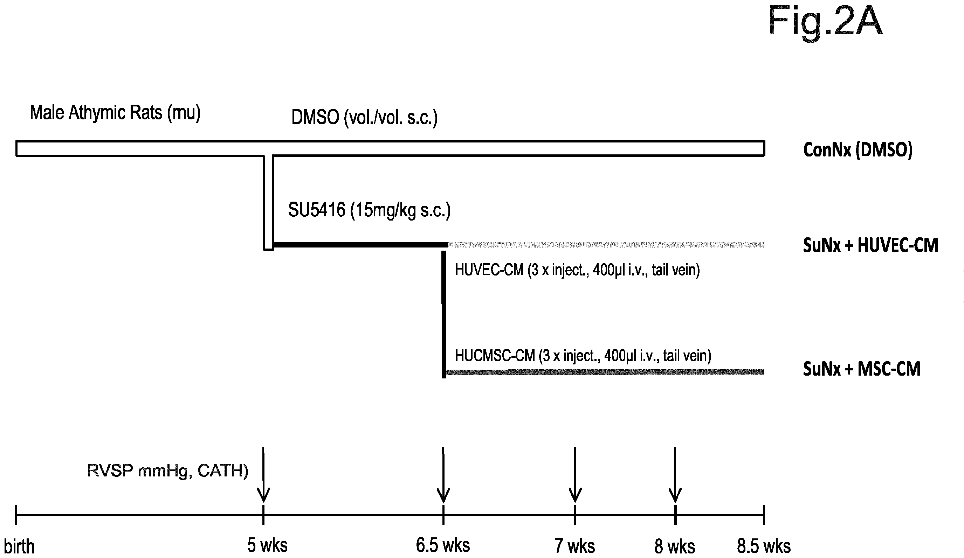

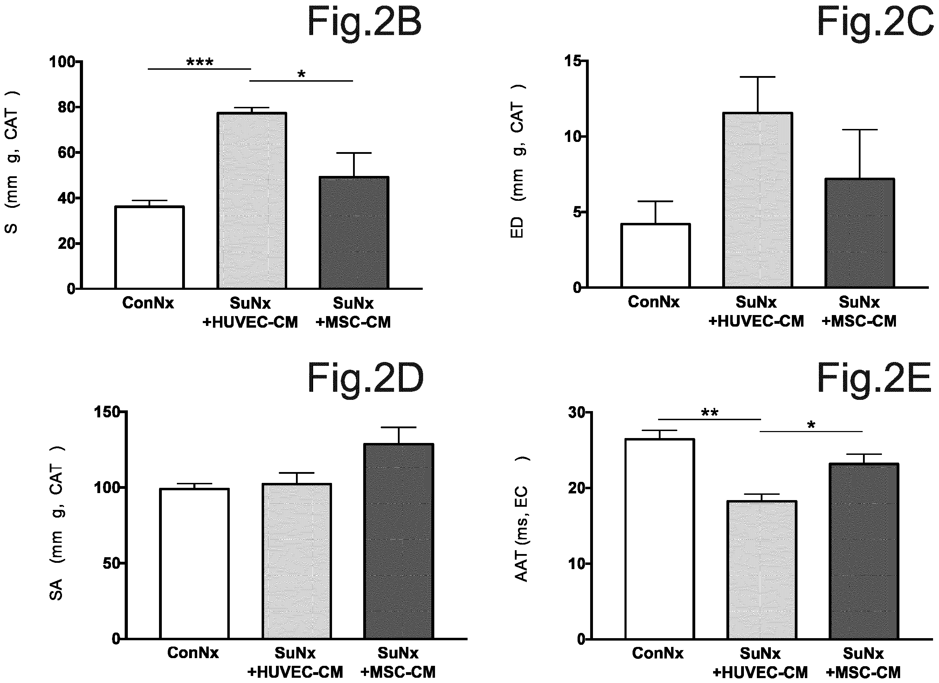

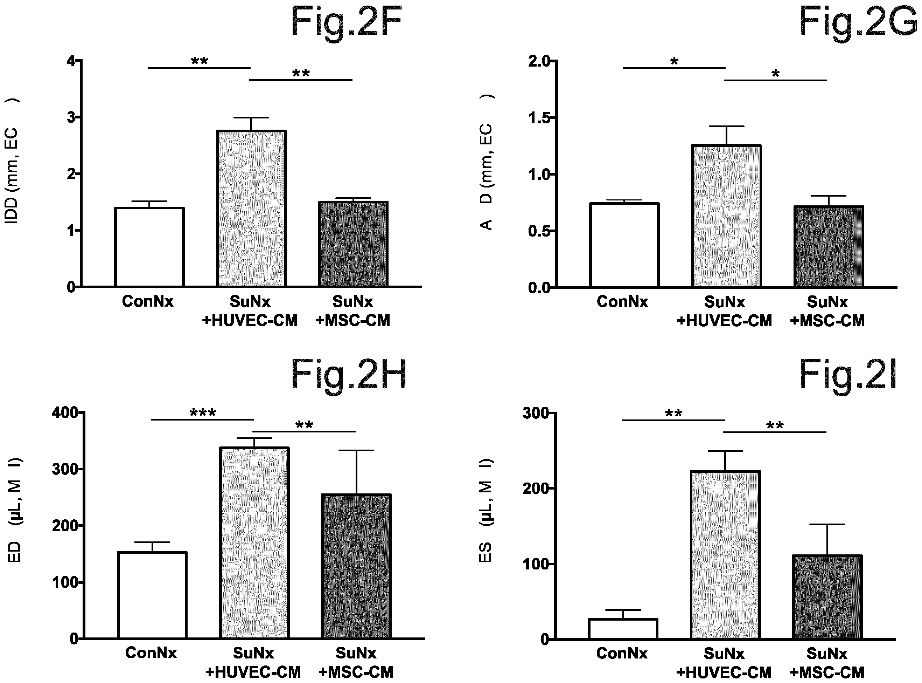

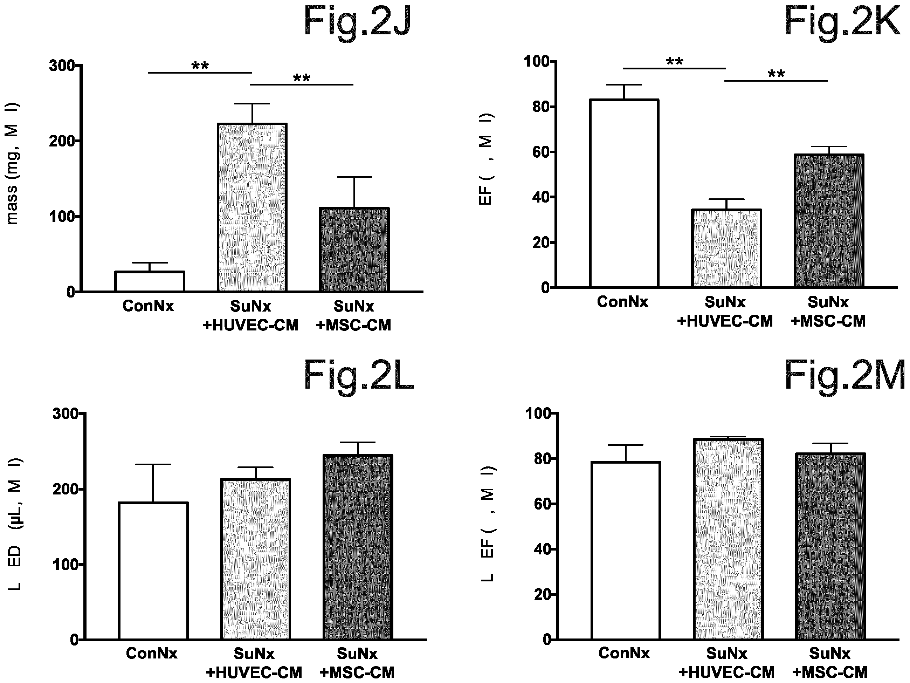

**Fig. 2****. Three intravenous injections of conditioned media derived from human umbilical cord mesenchymal stem cells reverse pulmonary arterial hypertension and prevent right ventricular failure in the Sugen-normoxia athymic rat model. (A)** Experimental design. Three age-matched groups: (i) ConNx [injected once subcutaneously with vehicle (DMSO; v/v)]; (ii) SuNx + HUVEC-CM [injected with SU5416 (20 mg/kg per dose, sc), and after 1.5 weeks injected with human umbilical vein endothelial cell conditioned media (3 injections)]; (iii) SuNx + MSC-CM [injected with SU5416 (20 mg/kg per dose, sc), and after 1.5 weeks injected with human mesenchymal stem cells conditioned media (3 injections)]. **(B-D)** Invasive hemodynamic measurements were performed through cannulation of the right jugular vein and right carotid artery (closed-chest) to assess the right ventricular systolic (RVSP), diastolic (RVEDP), systolic blood pressure (SAP). **(E-G)** Pulmonary artery acceleration time (PAAT) as a surrogate of mean PA pressure and pulmonary vascular resistance, end-diastolic diameter of the RV free wall (RVAWD), tricuspid annular peak systolic excursion (TAPSE) as a measure of longitudinal systolic RV function, were assessed via echocardiography. **(H-M)** Right ventricular end-diastolic (RV EDV), end-systolic volumes (RV ESV) and ejection fraction (RV EF) as a measure of RV dilation and systolic function, RV mass, LVEDV and LV EF were assessed by cardiac MRI. Mean ± SEM, n = 3 to 4, analysis of variance (ANOVA)-Bonferroni post hoc test, *P < 0.05, **P <0.01, ***P < 0.001.

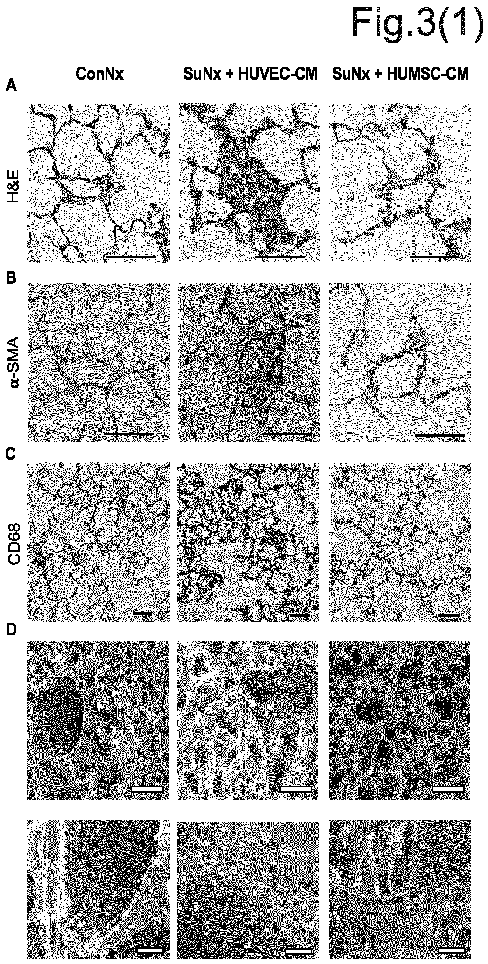

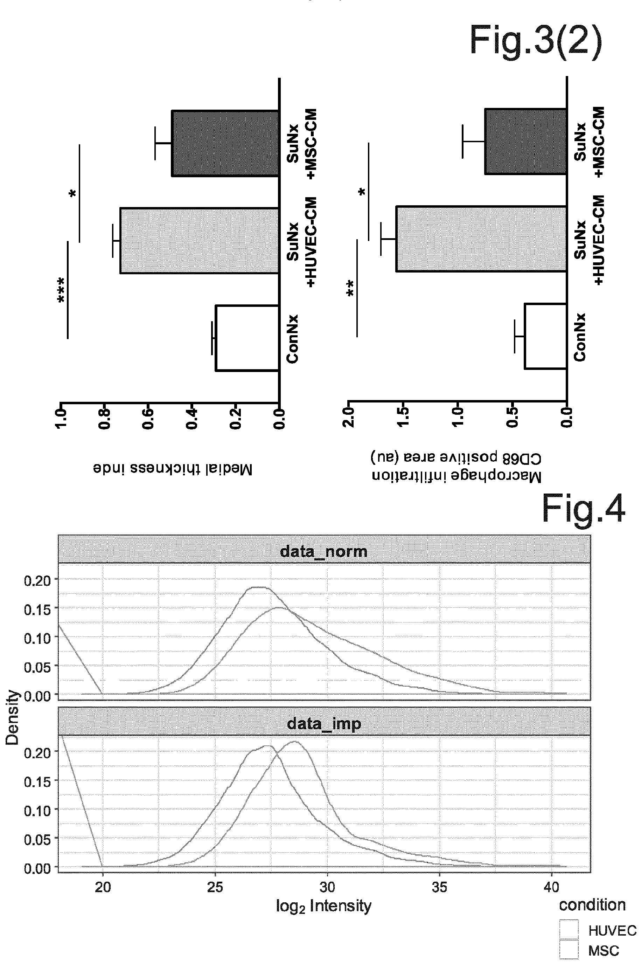

**Fig. 3****. Intravenous infusions of human umbilical cord MSC-conditioned media reverses obliterative pulmonary vascular remodeling and inflammation in the SuNx athymic rat model of PAH. (A)** Representative pictures of small, peripheral pulmonary arteries in H&E staining. Scale bar 50µm. **(B)** Representative images of α-SMA staining of small, peripheral pulmonary artery muscularization. Muscularization, represented as media thickness index, in pulmonary hypertensive rats was attenuated by MSC-CM, but not by HUVEC-CM administration. Scale bar 50µm. Mean±SEM, ANOVA-Bonferroni post-hoc-test, N = 3-5 indivudual animals 60 vessels/animal were counted, N=3-5 animals, *p<0.05, ***p<0.001. **(C)** Representative images of CD68 for infiltrating macrophages in the lung. Scale bar 100µm. Mean±SEM, N = 3-15 individual animals, 20-40 fields calculated, ANOVA-Bonferroni post-hoc-test, *p<0.05, **p<0.01. **(D)** Scanning electron micrographs illustrate the alveolar architecture of lungs. All lungs were harvested and processed immediately after the experiments described in Figure 1A. The lungs were freeze-dried and sputtered with gold in an argon atmosphere and examined using a Philips ESEM XL-30 scanning electron microscope at 15 keV and 21 µA (Philips, Eindhoven, Netherlands). High power images revealed thickened blood vessels (arrows) walls in SuNx lungs treated with HUVEC-CM, but not in control or MSC-CM treated lungs. Scale bars: 100 µm (upper row), 20 µm (lower row).
**Fig. 4****. Intensity data distribution before and after imputation shows that mean intensities slightly increased in both groups after imputation keeping roughly the same delta between the two means.** Average protein concentration is still higher in MSCs, even though MSC samples had a larger number of undetected (or possibly absent) proteins. Missing values are imputed using the Bayesian PCA missing value imputation.

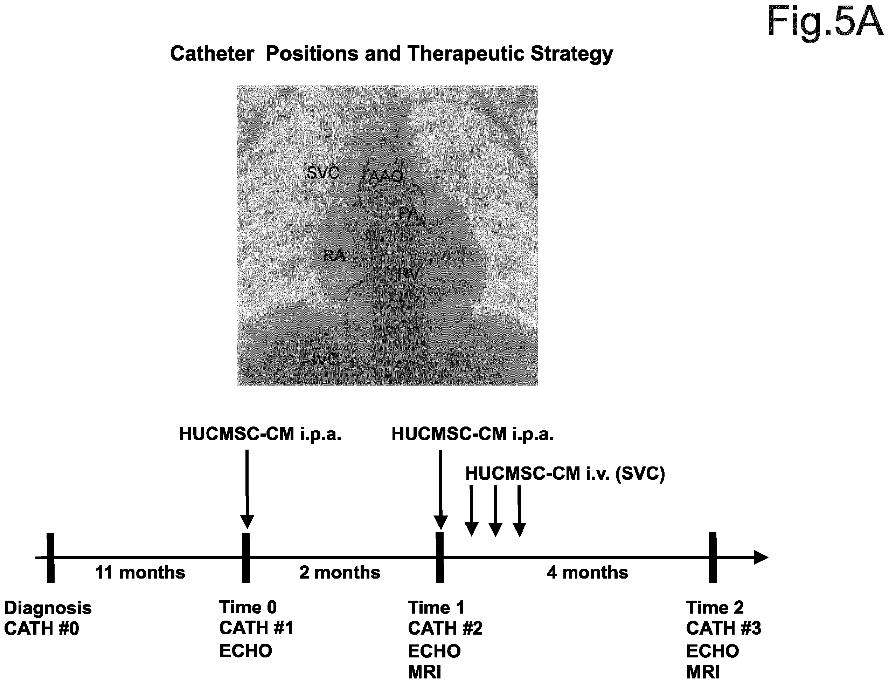

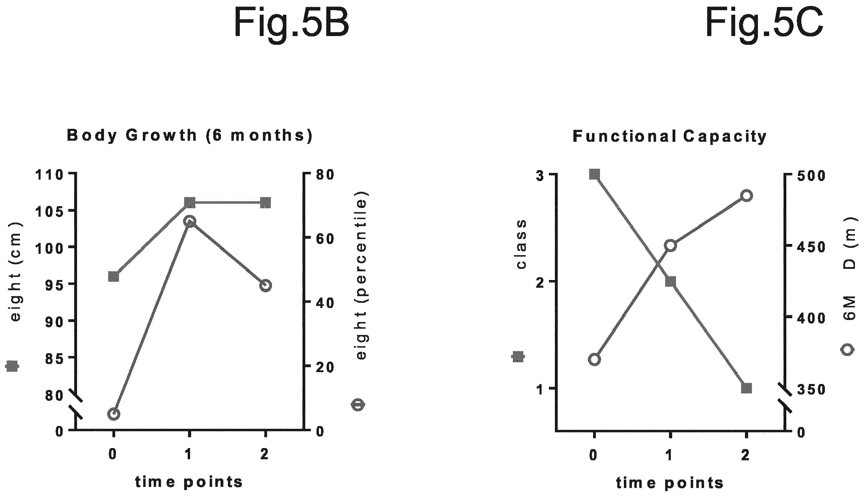

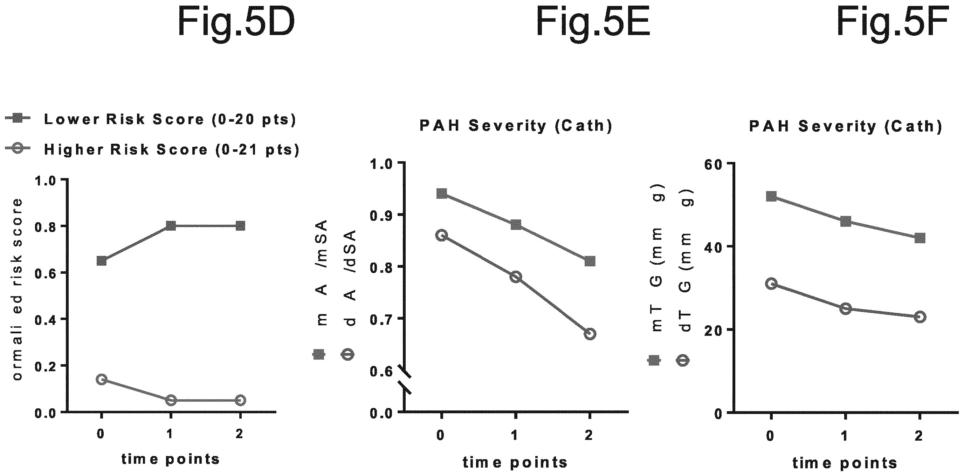

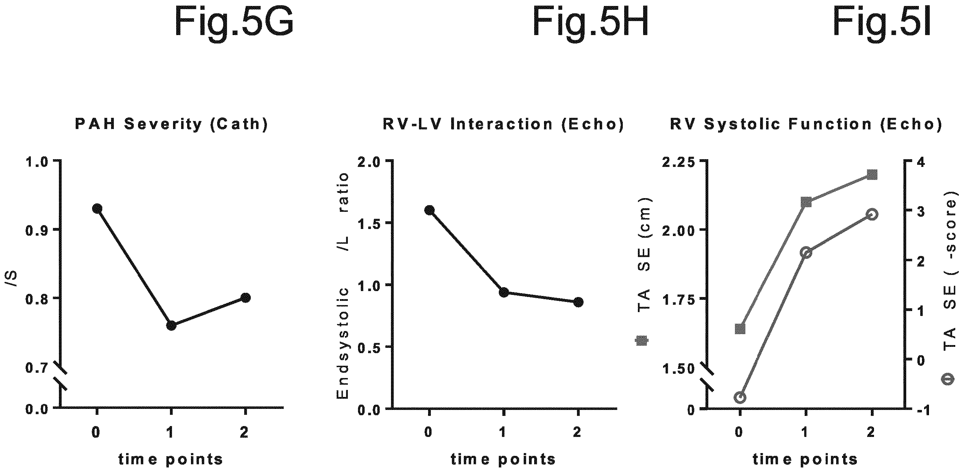

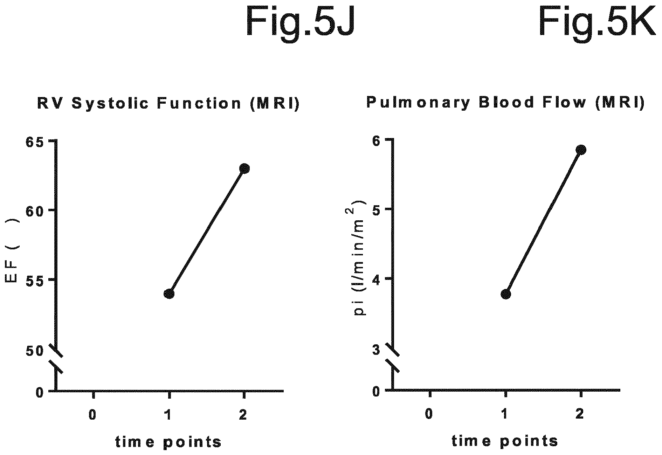

**Fig. 5****. Treatment of a 3year old child with severe heritable PAH with human umbilical cord mesenchymal stem cell-conditioned media (HUCMSC-CM) results in improvement of growth, functional capacity, risk scores, and multiple hemodynamic variables.**
   **(A)** Four catheterizations (CATH #0, 1, 2, 3) were performed spaced eleven, two- and four months apart (shown in the timeline as vertical bars). The PAH medication was not changed within the 6 months prior to CATH #1 (Time 0, at our institution), when for the first time, HUCMSC-CM was administered in the pulmonary arteries. The PAH medication was also not changed thereafter. During CATH #1 (Time 0) and CATH #2 (Time 1), after full invasive hemodynamic assessment, 200ml HUCMSC-CM was infused in the pulmonary arteries over one hour (100 ml into the right pulmonary artery over 30min.; 100 ml into the left pulmonary artery over 30min.) In the week following CATH #2 (Time 1), 200 ml HUCMSC-CM was infused via a central venous line on day 1, 2, and 3 after CATH #2. Improvements in body growth **(B),** functional capacity **(C),** EPPVDN risk scores **(D),** and key morphological and hemodynamic data **(E - K)** as assessed by cardiac catheterization, echocardiography and cardiac magnetic resonance imaging, were generated at the time points specified above (Time 0 - 2). See Supplementary information for methodological details. Abbreviations: 6MWD, 6-minute walking distance; AAO, ascending aorta; CATH, right and left heart catheterization; dTPG, diastolic transpulmonary pressure gradient; ECHO, transthoracic echocardiography; HUCMSC-CM, human umbilical cord mesenchymal stem cell-conditioned medialVC, inferior vena cava; LV, left ventricle; mPAP, mean pulmonary arterial pressure; MRI, cardiac magnetic resonance imaging; mSAP, mean systemic arterial pressure; mTPG, mean transpulmonary pressure gradient; PA, pulmonary artery; PVR, pulmonary vascular resistance; Qpi, pulmonary blood flow index; RA, right atrium; RV, right ventricle; RVEF, right ventricular ejection fraction; SVC, superior vena cava; SVR, systemic vascular resistance; TAPSE, tricuspid annular plane systolic excursion; WHO, world health organization.

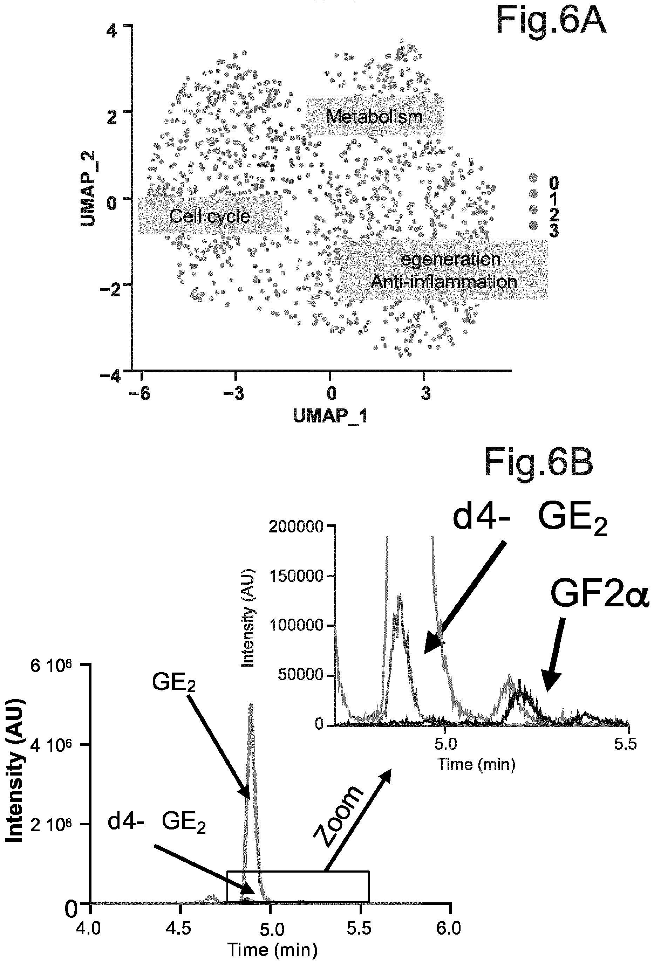

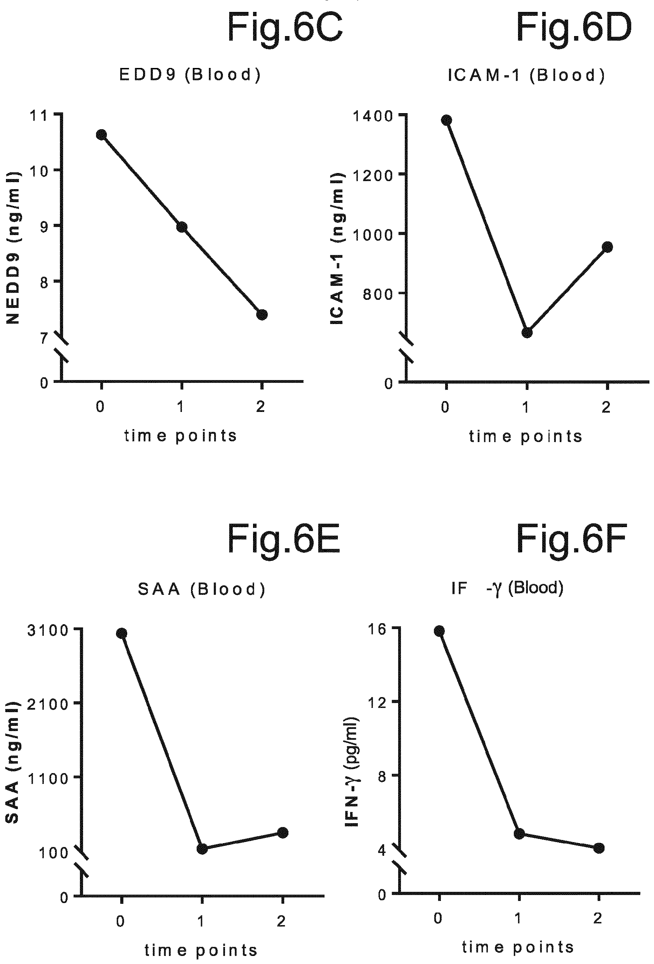

**Fig. 6****. Single-cell RNA sequencing of HUCMSC reveals four MSC clusters and a transcriptome enhanced for regeneration, anti-inflammation, cell cycle and metabolism.**
   **(A)** UMAP (Uniform Manifold Approximation and Projection) graph of the first two UMAP dimensions shows four clusters of MSC cells. Based on the pathway/GO annotation of their marker genes, the functional labels were added to the graph.
   MSC-like cells were isolated from the human umbilical cord (Wharton's jelly) following delivery of a full-term infant (38 weeks gestation). Following MSC culture, MSC were harvested in passage 3 to undergo scRNA-seq..
   **(B)** LC-MS chromatogram showing the mass spectrometric traces of PGE₂, PGF_{2α} and the internal standard d4-PGE₂.
   **(C-F)** EDTA plasma concentrations of NEDD-9, ICAM-1, SAA, and IFN-γ (mean of concentrations near-simultaneously measured in the SVC, IVC, and RA).

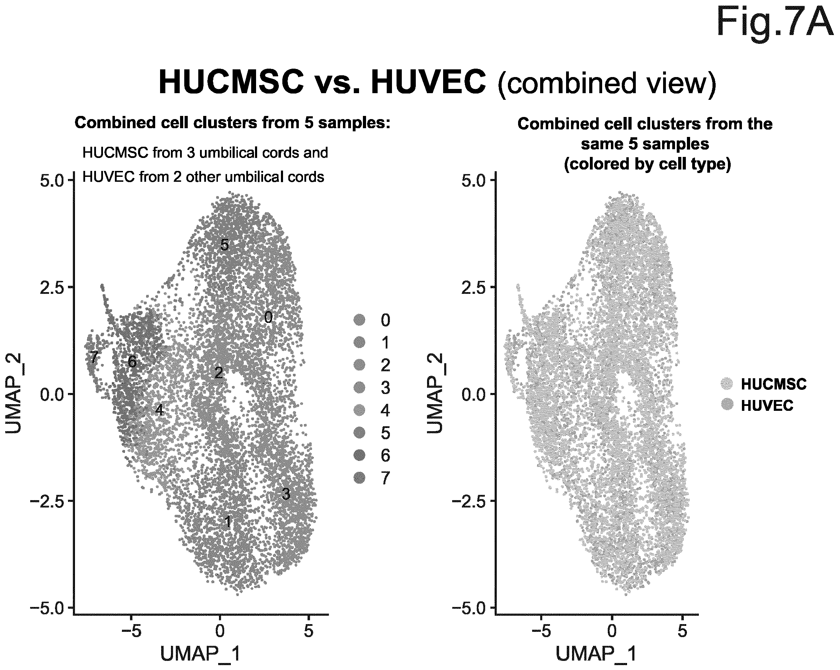

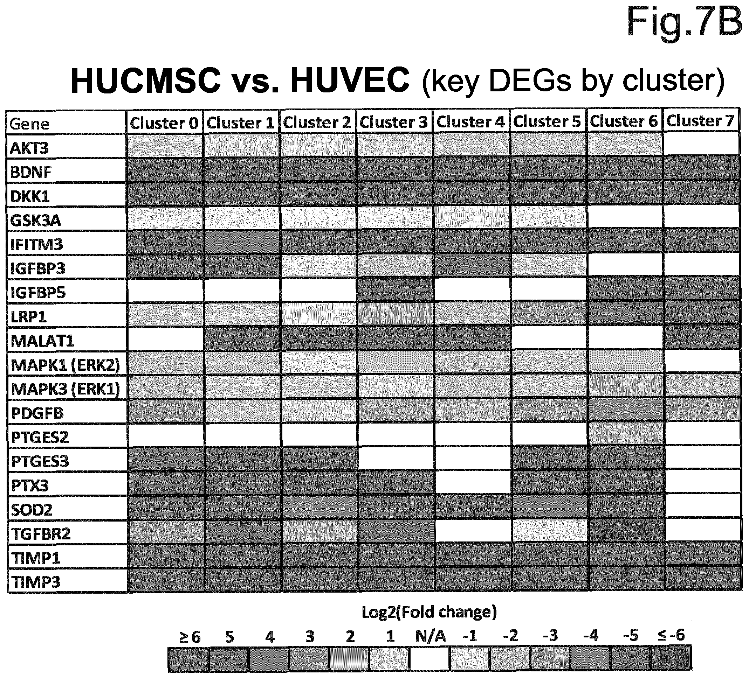

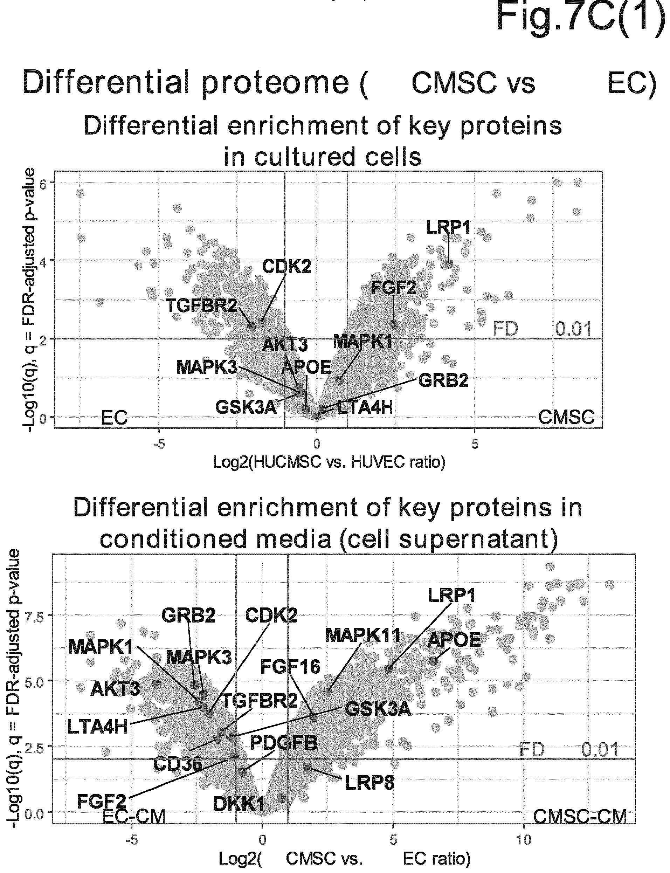

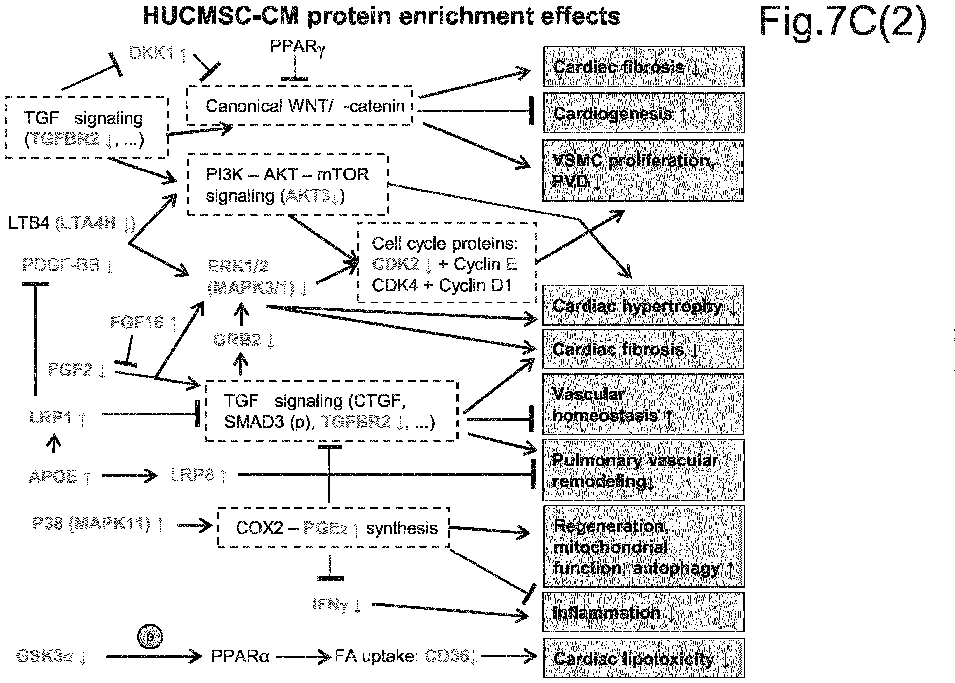

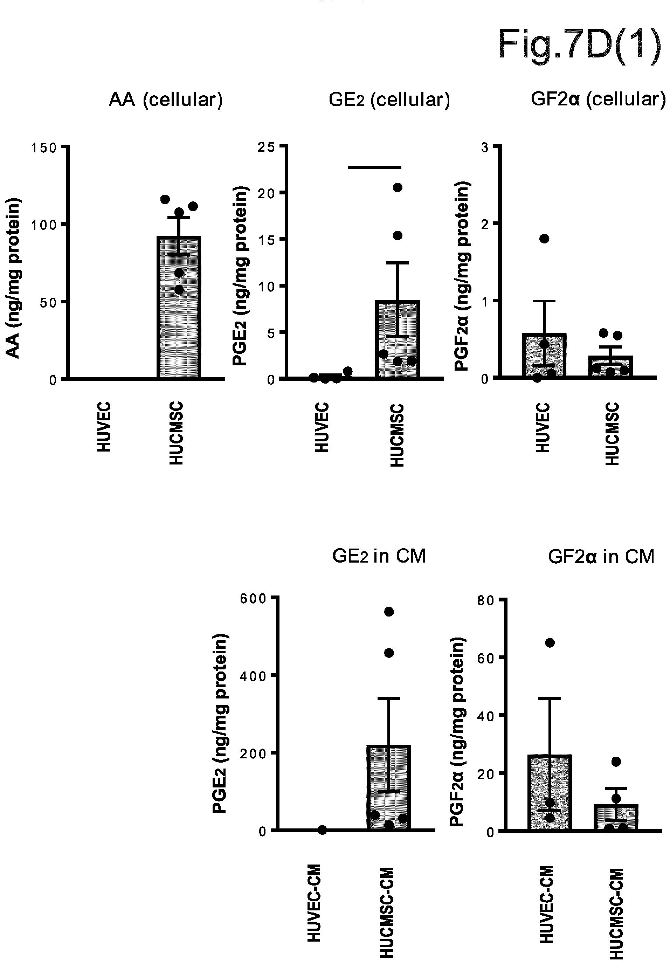

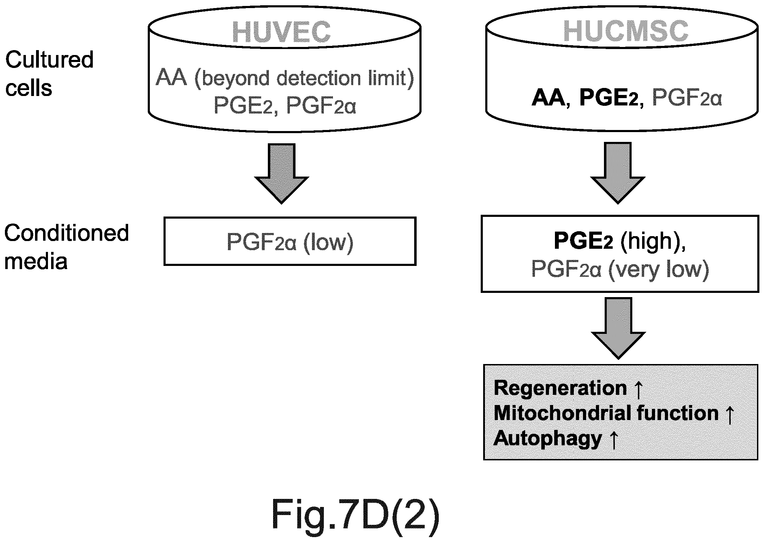

**Fig. 7****. Comprehensive analysis of cultured cells and conditioned media (single-cell transcriptome, proteome, and prostaglandins) reveals potential mechanisms explaining regenerative effects of HUCMSC conditioned media.**
   **(A)** Single-cell RNA expression analysis identified eight cell clusters with distinct expression profiles common to both HUCMSC and HUVEC cells. These clusters were used for differential gene expression analysis (HUCMSC vs. HUVEC). Sample sizes: HUCMSC n=3 (passage 6), HUVEC n=2 (passage 6).
   **(B)** The heat map of the key genes potentially contributing to regenerative effects of HUCMSCs in all eight clusters shown in panel (A). Selection of these genes was based on i) scRNA-seq analysis of the HUCMSCs whose conditioned media was used in treatment of the case, ii) results of the proteomics analysis shown in panel (C), and iii) association with synthesis of PGE₂ (the prostaglandin known for its regenerative capacity).
   **(C)** The volcano plots depict distribution of differentially enriched proteins (HUCMSC, n=5 vs. HUVEC, n=4). The key proteins relevant to the regenerative potential are shown as red dots. The proteins above the false discovery rate (FDR) threshold (<0.01) and passing the effect size threshold (0.5 > HUCMSC vs. HUVEC ratio > 2) were considered significant. Concentrations of conditioned media proteins deviated from their levels in cultured cells (in most cases increasing the degree of differential enrichment). On the right panel, we show potential effects of enrichment of the key proteins in the conditioned media. Proteins shown in green were observed in our data and correspond to the proteins labeled in the volcano plot (↑ = upregulation, ↓ = downregulation, (p) = phosphorylation, bold font = FDR-adjusted p-value < 0.01). Briefly, our data suggests that: 1) Downregulation of the TGFβ pathway suppresses canonical WNT signaling and thereby reduces cardiac fibrosis, promotes cardiogenesis, and inhibits VSMC proliferation; 2) Downregulation of GSK3α reduces FA uptake (manifested by CD36 downregulation) and reduces lipotoxicity; 3) Suppression of PI3K-AKT-mTOR and ERK1/2 signaling downregulates VSMC proliferation and cardiac hypertrophy; 4) Upregulation of P38 (MAPK11) promotes regenerative effects via induction of COX2-PGE₂ synthesis (also supported by prostaglandin analysis demonstrating high PGE₂ levels in HUCMSC but not in HUVEC both in cells and CM); 5) Suppression of TGFβ signaling via upregulation of APOE, LRP1, FGF16 and downregulation of TGFBR2, FGF2 reduces cardiac fibrosis and vascular homeostasis; 6) Suppression of ERK1/2 signaling reduces VSMC proliferation; 7) Upregulation of the APOE-LRP8 cascade suppresses pulmonary vascular remodeling.
   **(D)** LC-MS prostaglandin analysis revealed that the primary difference between HUCMSC (n=5) and HUVEC (n=4) samples was in the significantly higher HUCMSC levels of PGE₂ (both in cells and conditioned media) and higher levels of arachidonic acid (AA) in HUCMSC cells, while the HUVEC AA levels were below the detection limit. Since AA is a precursor of PGE₂, the upregulation of PGE₂ in HUCMSC is likely associated with the higher HUCMSC AA level. Importantly, PGE₂ greatly contributes to the paracrine regenerative and immunomodulatory effects of MSCs in preclinical in vivo and in vitro studies (for example, PGE₂-induced suppression of IFN-gamma and TGFβ signals). Trace amounts of PGF_{2α} were also detected without significant enrichment in either of the groups. The values in bar plots values are expressed as mean ± SEM; two-tailed Mann Whitney U test was used (*p < 0.05, **p < 0.01).

### Examples

### Example 1

The inventors decided to administer HUCMSC-conditioned media intravascularily (intravenously, intra pulmonary artery) to subjects, animals and human individuals, with chronic progressive heart-lung and vascular disease. While genetically engineered mouse models are very helpful in identifying the possible relevance of emerging signaling pathways in vivo, the hemodynamic and morphological cardiovascular pathobiology of human PAH and other chronic, progressive, debilitating heart-lung and vascular diseases is better resembled in heart and lungs of rats, or larger animals. Thus, any new therapeutic intervention should be tested in such models that closely simulate human disease before setting up clinical phase 1 or phase 2 PAH studies.

Thus, the inventors used the SuNx athymic rat as a model for severe, progressive PAH with fatal RV failure, and demonstrated that three subsequent intravenous injections of HUCMSC-CM greatly improved pulmonary arterial pressure and systolic RV function, reduced RV mass to control level, and prevented fatal RV failure. PGE₂ and several proteins were identified to be the likely most beneficial ingredients of then HUCMSC-CM. HUCMSC-CM was found to have very high anti-inflammatory, vasodilatory, regenerative/pro-angiogenic and reverse-remodeling properties.

### Preparation of human umbilical cord mesenchymal stem cells

Human umbilical cord-derived MSC (HUCMSC) were isolated from individual either term-deliveries (38-40 weeks) or by Cesarean section patients after obtaining informed written consent, respectively, as approved by the Institutional Review Board.

First, blood from arteries and the vein was removed by flushing PBS through the vessels using a sterile syringe and blunt-end needles. The umbilical cord tissue was cut into 10-15 cm long segments which were subsequently cut into approx. 0.5 cm³ large tissue pieces. The pieces were transferred to cell culture flasks and incubated by high stringency in αMEM supplemented with 10% of allogenic human serum, 100 U/ml penicillin, 100 µg/ml streptomycin and 2mM L-glutamine at 37°C in a humidified atmosphere with 5% CO₂. The medium was changed every second day. Following outgrowth of adherent cells from single tissue pieces, the umbilical cord tissue was removed and, at about 80% of confluency, the adherent cells were harvested by accutase treatment. They were plated at a density of 3,000 cells/cm² in cell culture flasks.

The isolated populations were extensively characterized as mesenchymal stem cells by surface marker analysis and functional properties including proliferative capacity. In this context, the International Society for Cellular Therapy (ISCT) proposed in 2006 the minimal criteria defining these cells as "Multipotent Mesenchymal Stromal/Stem-like Cells" (MSC) by the expression of CD73, CD90, and CD105 with concomitant absence of at least CD14, CD31, CD34, and CD45 surface marker molecules, as well as presence of detectable G1, S, and G2/M phases. These criteria were met by the isolated cells.

HUC-derived cells could be efficiently cryopreserved and revitalized. A cryo-medium containing 90% of allogenic human serum and 10 % (v/v) DMSO was used. The cells were gradually frozen at a rate of 1°C/min and finally stored at -196°C in liquid nitrogen.

A method for preparation of human umbilical cord mesenchymal stem cells and MSC-conditioned media is also shown in Fig. 1.

### Preparation of human umbilical vein-derived endothelial cells (HUVECs)

Human umbilical vein-derived endothelial cells (HUVECs) from different donors were isolated by enzymatic digestion and flushing through the umbilical vein using a sterile syringe with a blunt-end needle. Isolated cells were cultured by high stringency in endothelial cell basal medium MV2 (PromoCell GmbH, Heidelberg, Germany) together with the endothelial cell growth medium supplement mix (PromoCell GmbH). Subculture of HUVECs was performed by treatment with Trypsin/EDTA solution (Sigma) for 10 min at 37°C. Following characterization of these cells by determination the expression of endothelial markers (e.g. CD31), HUVECs could be cryo-preserved in 80% FCS, 10% (v/v) culture medium, and 10% (v/v) DMSO. The cells were gradually frozen at a rate of 1°C/min and finally stored at -196°C in liquid nitrogen.

### Conditioned medium (CM) collection of HUCMSC and HUVEC.

Following MSC culture in sub-confluent growth phase, serumfree medium supernatant was harvested as conditioned medium after 36h, centrifuged (3.185g / 10min) and cryo-preserved.

Alternatively, a 10-fold concentrated supernatant was obtained by using an Amicon Ultra4 chamber (10 kDa membrane, Amicon Ultracel, Merck-Millipore, Darmstadt, Germany) according to the manufacturer's recommendations and aliquots were similarly cryo-preserved.

### The SuNx athymic rat animal model

The major, shared features of the immunocompetent Sugen-hypoxia (SuHx) and the athymic Sugen normoxia (SuNx) rat models are severe PAH, obliterative pulmonary vascular remodeling, severe RV dysfunction, and broad treatment resistance, in the absence of parenchymal lung disease/emphysema - as is the case in human idiopathic or heritable PAH (IPAH/HPAH). The additional hallmark specific to the SuNx athymic rat PAH model is the absence of T lymphocytes, in particular, vasoprotective T regulatory cells, a high degree of macrophage-driven inflammation, RV coronary vascular rarefaction, and very rapid progression to heart failure and death within 1-5 weeks of VEGFR2 blockade by SU5416.

Five week old male athymic (rnu) rats weighing ≈100g were purchased from Charles River. These so-called "nude" rats have no T-cells, in particular, no regulatory T cells, as the culprit of the severe PAH-phenotype and high mortality even in normoxia. On the other hand, there was no relevant immunological reaction to human conditioned medium in these athymic rats. Rats were divided into 3 age-matched groups, according to the experimental design **(****Fig. 2A**): (1) control normoxia (ConNx), i.e. rats injected once subcutaneously with vehicle (DMSO; vol./vol.), (2) Sugen normoxia (SuNx), i.e. rats injected with the VEGFR2 inhibitor SU5416 (Sigma), 15mg/kg/dose subcutaneously dissolved in DMSO, treated three times with HUVEC-CM (10, 14 and 21 days after SU5416 injection), (3) Sugen normoxia (SuNx), i.e. rats injected with the VEGFR2 inhibitor SU5416 (Sigma), 15mg/kg/dose subcutaneously dissolved in DMSO, treated three times with HUCMSC-CM. Dose finding studies 10, 15 and 20mg SU5416/kg body weight/dose had been performed.

*Animal phenotyping.* Cardiac catheterization (RV, LV; closed chest), echocardiography, MR cine imaging (mass and volumes), MR spectroscopy (intramyocellular lipid content), organ harvest, histology, immunohistochemistry and electron microscopy were performed as described in Legchenko et al, 2018. Briefly, transthoracic echocardiography was performed at age 6.5 weeks and 8 weeks following a standardized protocol (i.e. 10 and 21 days after subcutaneous SU5416 injection). The rats also underwent cardiac magnetic tomography at the age of 8 weeks for the assessment of ventricular mass and volumes as well as biventricular systolic function, as previously described. At the age of 8.5 weeks (days 24-27 after SU5416), surviving, spontaneously breathing rats underwent closed-chest right and left heart catheterization, followed by heart and lung harvest, as previously described.

The inventors evaluated the impact of human umbilical cord-derived MSC-conditioned media (HUCMSC-CM) on the progression of PAH and right heart failure in the normoxic Sugen athymic rat model (SuNx). They found that regularly concentrated HUCMSC-CM was at least as effective as 10x highly-concentrated HUCMSC-CM (filtered over a 10kDa membrane), indicating that the low molecular weight and size components (e.g. small proteins, peptides, metabolites, oxylipids, RNA, EVs) are mainly responsible for the beneficial effects of MSC-CM rather than high molecular weight components. Thus, regularly concentrated, non-filtered CM were used for the experiments. Three intravenous doses of HUCMSC-CM led to reversal of PAH and prevention of RV Failure I the athymic SuNx rats **(****Fig. 2****).**

Subsequently, oxylipd analysis and proteomics analysis of the HUCMSC-CM and HUVEC-CM **(****Fig. 3****)** were performed to better characterize the active and beneficial ingredients of the HUCMSC-CM that led to PAH reversal and prevention of RV failure in the SuNx athymic rat.

### RESULTS

***In vivo:*** HUCMSC-CM treated SuNx athymic rats had lower RVSP than HUVEC-CM-treated rats (49.3±10.5 mmHg in SuNx+HUCMSC-CM vs. 77.4±2.3 mmHg in SuNx+HUVEC-CM), that was close to control level (36.2±2.7 mmHg in ConNx; p < 0.001). There were no differences in systemic blood pressure between the groups. Additional experiments were performed by applying echocardiography and cardiac MRI: The pulmonary artery acceleration time (PAAT) is inversely associated with pulmonary arterial pressure (PAP) and pulmonary vascular resistance (PVR), and improved with HUCMSC-CM treatment (26.4±1.2 ms in ConNx vs. 18.29±0.9ms in SuNx+HUVEC-CM vs 21.2±1.3ms in SuNx+HUCMSC-CM; p<0.05). RV end-diastolic inner diameter (1.4±0.1 mm vs 2.8±0.2mm vs 1.5±0.1mm) and RV anterior wall thickness (0.74±0.03 mm vs 1.26±0.17mm vs 0.72±0.1mm) decreased with HUCMSC-CM treatment. Cardiac MRI demonstrated prominent improvement in RV ejection fraction (83.1±6.6% vs 34.5±4.6% vs 58.7±3.7%; p<0.05) and decrease in RV mass to control level (123.0±6.9mg 343.7±29.8mg vs 146.0±4.7mg; p<0.001) in SuNx+HUCMSC-CM rats.

There has not been a single stem cell-related intervention published that had been demonstrated to be as efficient in a rat model of PAH/RV failure, and certainly no stem-cell-derived intervention has been shown to be effective at all in this most aggressive rat model, i.e. the SuNx athymic rat.

Moreover, intravenous infusions of HUCMSC-CM reverses pulmonary vascular remolding and pulmonary inflammation in the athymic SuNx rat model **(****Fig. 3****).**

***Ex vivo:*** By applying oxylipid analysis and proteomic analysis ex vivo, the inventors identified PGE₂ and several (up to 10) proteins and peptides to be highly upregulated (>> 10 fold) in HUCMSC-CM vs. HUVEC-CM (data not shown). The intensity distribution of the proteomics data are shown in **Figure 4****,** illustrating the major differences in the proteome signature between HUCMSC-CM and HUVEC-CM. Additional indepth caharacterization is shown in **Fig.6****.**

### Example 2

The inventors demonstrate safe and efficient human umbilical cord mesenchymal stem cell (HUCMSC)-derived treatment of severe, progressive PAH, by means of serial intravascular infusions of HUCMSC-conditioned media in one young patient with heritable PAH and HHT type 2 caused by a *ACVLR1* missense mutation.

### RESULTS

At diagnosis, the 3-year-old girl was in critical condition, status post two syncopal, "afebrile seizure episodes", in WHO functional class 4, with a 6 minute-walking-distance of only 270 meters (SpO₂>95%), and moderate thrombocytopenia at 80.10³/mcL. She had a 10-months history of fatigue, repetitive nose bleeding (epistaxis), and mucocutaneous telangiectases at the lips, chest and lower extremities. Serum NTproBNP was greatly elevated at 2,414 ng/L. Echocardiography showed severely compromised right ventricular (RV) systolic function (TAPSE 1.4 cm) and tricuspid regurgitation, grade 2. The first diagnostic cardiac catheterization (CATH #0; treatment naive) was conducted in January 2019 at an external tertiary center. Invasive hemodynamic measurements demonstrated severe suprasystemic PAH (pressures: PA 119/57/85 mmHg, AAO 94/43/63 mmHg, mPAP/mSAP ratio 1.35), severely elevated pulmonary vascular resistance (PVRi 21 WU*m²; PVR/SVR ratio 1.2), lack of acute vasoreactivity (AVT), and normal cardiac index (Qsi 3.6 L/min/m²). Accordingly, at diagnosis, the patient reached "higher-risk" stratification, with a European Pediatric PVD Network (EPPVDN) higher risk score of up to 0.8 (12/15) and lower risk score down to 0. The ECG was consistent with tachycardic sinus rhythm, right atrial enlargement, RV hypertrophy and strain. Chest X-ray and CT showed severe dilation of the RV and PAs, but normal lung parenchyma, no evidence for thrombi or veno-occlusive disease. Pulmonary angiograms demonstrated an abnormal peripheral pulmonary vascular pattern characterized by very prominent arterial tortuosity and haziness of the contrast dye in the peripheral pulmonary circulation; the latter may represent diffuse (pre)capillary telangiectasia and/or very small arterio-venous malformations throughout (data not shown). According to Curaçao's diagnostic criteria for hereditary hemorrhagic telangiectasia (HHT, Osler-Weber-Rendu syndrome), as laid down ino international guidelines (2000, 2020) Shovlin et al., 2000; Faughnan et al., 2011), she had HHT.

The patient was found to have a heterozygous missense mutation in the *ACVRL1* gene c.1451G>A, p.(Arg484Gln). This variant occurred *de novo* and has been previously reported in seven patients with either isolated PAH or PAH plus HHT, qualifying her to have heritable PAH and HHT type 2.

After diagnosis (CATH #0), the patient was started on dual oral combination therapy (sildenafil, bosentan), and referred to Hannover Medical School for lung transplant evaluation. After limited response to initial dual oral therapy, the PAH-targeted pharmacotherapy was modified to include oral sildenafil, macitentan, and spironolactone, and inhalative iloprost. Since the systemic blood pressure remained borderline low (systolic 80 mmHg), we did not start intravenous prostacyclin analogs. Under these measures, the patient was clinically stabilized and the WHO functional class and 6-minute-walk distance improved (data not shown).

Nevertheless, echocardiographic variables of RV-LV-Interaction and LV underfilling (RV/LV end-systolic ratio, LV end-systolic eccentricity index), and pulmonary artery acceleration time (an inverse surrogate of PAH severity) were still greatly abnormal. Global and longitudinal systolic RV function was reduced. Owing to the grim prognosis, a consented compassionate use of allogenic human umbilical cord MSC-derived therapy was performed.

To this end, the inventors isolated MSC-like cells (HUCMSCs) (Lavrentieva et al., 2010) from the patient's younger sibling's umbilical cord and collected conditioned media (CM) (see **Methods).** The PAH patient received a series of 5 non-GMP-certified, allogenic HUCMSC-CM infusions over 6 months during two hospital stays (Time 0, Time 1), via an intrapulmonary arterial catheter and a central venous catheter. All HUCMSC-CM infusions were tolerated very well. Serum CRP and IL-6 levels remained normal, and the patient did not receive any antibiotic, anti-allergic or anti-inflammatory medication. Post-infusion monitoring was at a minimum 24 hours, followed by close outpatient care every 1-6 weeks. Clinical status and hemodynamics were assessed at baseline (Time 0, CATH #1), after two (Time 1, CATH #2) and six months (Time 2, CATH #3; **Figure 5A****).**

In the interval between diagnosis and the start of therapeutic HUCMSC-CM intervention, there was essentially no weight gain and no growth (height) in 12 months. After the first HUCMSC-CM infusion **(****Figure 5A****),** the patient started to grow: +10 cm length in 3 months (gain from the 5^{th} to the 65^{th} percentile, **Figure 5B****).** Moreover, cardiopulmonary exercise capacity greatly increased, as judged by WHO functional class (from 3 to 1) and 6-minute-walk distance (from 370 to 485m; **Figure 5C****).** The girl is now 6 years old and doing very well, without any limitations in exercise capacity.

Consistent with the clinical improvements, the better EPPVDN risk scores **(****Figure 5D****),** cardiac catheterization **(**Figure 5E-G) and echocardiography **(**Figure 5 H-I) data confirmed the beneficial effect of HUCMSC-CM treatment: PA pressure (PAP) and PAP gradients **(****Figure 5E**, 5F) as well as PVR/SVR ratio **(****Figure 5G****)** decreased by 14-26% **(**Figure 5 E-G), indicating a marked decrease in PAH severity. In addition, echocardiography demonstrated that RV-LV interaction as judged by normalized end-systolic RV/LV ratio **(****Figure 5H****),** and RV systolic function (TAPSE, **Figure 5I**), greatly improved. Normalization of systolic RV function and pulmonary blood flow were confirmed by cardiac MRI at Time 1 and 2 **(****Figure 5J,** 5K).

To explore the possible mechanisms of HUCMSC-derived therapy the inventors performed single-cell RNA sequencing of the subcultured MSCs used to harvest CM from for the compassionate use treatment **(****Figure 6A****),** mass spectrometry of MSC-CM **(****Figure 6B****),** and protein expression assays of the patient's blood plasma over time **(**Figure 6C-F). Single-cell RNA sequencing of the subcultured HUCMSCs identified four functionally different cell subpopulations (clusters). The four MSC subpopulations are visualized in **Figure 6A** with functional labels, based on the pathway/GO annotation of their marker genes (clusters 0-3; **Figure 6A****).** An expression heatmap (data not shown) shows of three sets of upregulated genes whose expression separates the cells into the subpopulations (top 10 per cluster shown; no upregulated genes for cluster 3).

Particularly, cell cluster 0 enhanced the transcriptome for regeneration and anti-inflammation, and likely secretes molecules whose paracrine effects provide beneficial effects on right heart-pulmonary circulation.

The inventors hypothesized that boosted prostaglandin E₂ (PGE₂) production may be a major regenerative and immunomodulatory component (Hass et al., 2011) in HUCMSC-CM. Indeed, the scRNA-seq data showed that the genes encoding two PGE₂ synthesis enzymes (*PTGES2* and *PTGES3),* as well as *PTGS2* (COX2; a gene involved in conversion of arachidonic acid into PGH₂ required for production of PGE₂), were expressed in the majority of HUCMSCs, as opposed to the enzymes that synthesize PGI₂ and PGD₂ (*PTGIS* and *PTGDS* faintly expressed only in a few cells; data not shown). Consistent with the inventors' scRNA-seq data, the analysis of the HUCMSC-CM prostaglandins detected a major PGE₂ signal, but only minimal levels of PGF_{2α} **(****Figure 6B****)** and no PGD₂, altogether suggesting that HUCMSC-CM-secreted PGE₂ may contribute to the beneficial effect of the HUCMSC-CM on the PAH patient.

HUCMSC-derived therapy decreased patient blood plasma markers of vascular (endothelial) fibrosis (NEDD9) (Samokhin et al., 2018), vascular injury (ICAM-1) and inflammation (SAA; IFN-γ) **(**Figure 6C-F). These results are consistent with the inventors' r scRNA-seq data (PGE₂ synthesis genes) and the subsequent validation in cultured cells and conditioned media (single-cell transcriptome, proteome, and prostaglandins) from multiple umbilical cords, described below **(****Figure 7****).**

The inventors expanded their single-cell RNA expression analysis to include HUCMSCs (3 umbilical cords) and HUVEC controls (2 umbilical cords), and identified eight cell clusters with distinct expression profiles **(****Figure 7A****).**

Based on analysis of differentially expressed genes in these eight clusters (HUCMSC vs. HUVEC; data not shown), the inventors observed that - in general - all eight clusters had expression profiles confirming the beneficial role of HUCMSCs, e.g., synthesis of PGE2 and many secreted proteins (e.g., DKK1, LRP1, TGFBR2) known for their role in regenerative pathways **(****Figure 7B****).** Concentrations of conditioned media proteins deviated from their intracellular levels in cultured cells in a way that in most cases increased the degree of differential enrichment (Figure 7C).

Analysis of prostaglandins, including the precursor arachidonic acid (AA) (Figure 7D), demonstrated much higher levels of HUCMSC PGE₂ (both in cells and CM) and higher levels of AA in HUCMSC versus HUVECs (HUVEC AA levels were below the detection limit). Since AA is a precursor of PGE₂, the upregulation of PGE₂ in HUCMSCs is likely associated with the higher HUCMSC AA level. Trace amounts of PGF_{2α} were also detected without significant enrichment in either of the groups. Taken together, LC-MS analysis identified very high levels of PGE₂ in HUCMSCs and HUCMSC-CM but not in HUVECs or HUVEC-CM (Figure 7D), most likely due to boosted AA-PGE₂ synthesis in HUCMSCs.

### DISCUSSION

The inventors reported the first-in-human application of umbilical cord mesenchymal stem cell-derived conditioned media (HUCMSC-CM) to treat severe, progressive PAH in a patient (Hansmann et al., 2022, in press). Serial infusions of HUCMSC-CM resulted in marked clinical and hemodynamic improvement after 6 months, and showed no adverse events. The HUCMSC transcriptome (from 3 umbilical cords, unrelated donors) suggested enhancement of regeneration, mitochondrial function, autophagy, and anti-inflammation pathways. Proteomics analysis revealed that the proteins differentially enriched in HUCMSC-CM modulate several key pathways to: i) reduce cardiac fibrosis and hypertrophy, VSMC proliferation, pulmonary vascular remodeling, inflammation, and cardiac lipotoxicity, and ii) increase cardiogenesis, vascular homeostasis, regeneration, mitochondrial function, and autophagy. Analysis of prostaglandins and AA showed boosted paracrine PGE₂ signaling derived from cellular AA metabolism in HUCMSCs.

Importantly, HUCMSC-derived therapy decreased established blood plasma markers of vascular (endothelial) fibrosis (NEDD9) (Samokhin et al., 2018), vascular injury (ICAM-1) and inflammation (SAA; IFN-γ) in this patient. NEDD9 targets collagen type 3 A1 and promotes endothelial fibrosis in experimental PAH (Samokhin et al., 2018). Moreover, NEDD9 interacts with P-selectin and drives detrimental platelet-endothelial adhesion in the pulmonary circulation (Alba et al., 2021). Endothelial *NEDD9* expression is regulated by aldosterone (independently of *TGFβ* signals) (Samokhin et al., 2018) and increased in fibrotic arterioles of PAH patients (Samokhin et al., 2018). Blood plasma NEDD9 has been shown to be increased in adult PAH by 1.8-fold and to correlate positively with prognostic variables (PVR), and negatively with RV function (RVEF), exercise capacity (6MWD) and lung transplant-free survival (Samokhin et al., 2020). Importantly, NEDD9 inhibition prevented experimental PAH (Samokhin et al., 2018)..

The inventors previously demonstrated that aldosterone, which regulates *NEDD9* in endothelial cells (Samokhin et al., 2018), increases with PAH severity in the blood plasma of adults (Calvier et al., 2016). Here, HUCMSC-CM treatment, particularly the first dose, decreased the circulating vascular injury marker ICAM-1 that is elevated in PAH (Calvier et al., 2016), and the pro-inflammatory mediators serum amyloid A (SAA) and IFN-γ, besides *NEDD9.*

The inventors validated the case findings in a subsequent multiple-cord omics analysis of HUCMSCs vs. HUVECs, and their secretome (CM): at the molecular level, they confirmed upregulation and predicted secretion of the key regeneration/proliferation molecules: *IGFBP3, IGFBP5, BDNF, TIMP1,* and *TIMP3* (upregulated in the regenerative cell cluster, i.e., Cluster 0, of the scRNA-seq results of the treated patient), which were also found as upregulated in most clusters of the integrated scRNA-seq analysis (HUCMSCs vs. HUVECs; **Figure 7B****)** and positively enriched in HUCMSC-CM proteins. Moreover, the results of the proteomics analysis presented in Figure 7C suggested a variety of mechanisms that likely contribute to improvements of the cardiovascular function in the patient. Briefly, the inventors' multiple-cord data analysis suggests that: 1) Downregulation of the *TGFβ* pathway suppresses canonical WNT signaling and thereby reduces cardiac fibrosis (Akhmetshina et al., 2012), promotes cardiogenesis, and inhibits VSMC proliferation (Tsaousi et al., 2011); 2) Downregulation of GSK3α reduces FA uptake (manifested by CD36 downregulation) and reduces lipotoxicity (Nakamura et al., 2019); 3) Suppression of PI3K-AKT-mTOR and ERK1/2 signaling (Hansmann et al., 2008) downregulates VSMC proliferation and cardiac hypertrophy; 4) Upregulation of P38 (MAPK11) promotes regenerative effects via induction of COX2-PGE₂ synthesis (Yu et al., 2014; North et al., 2007) (also supported by LC-MS analysis of prostaglandins demonstrating high PGE₂ levels in HUCMSC but not in HUVEC both in cells and CM (Figure 7D)); 5) Upregulation of APOE (Hansmann et al., 2007), LRP1 (Calvier et al., 2019), FGF16 (Fujiu et al., 2014) and downregulation of TGFBR2 and FGF2 (Fujiu et al., 2014) suppresses TGFβ signaling, thereby reducing cardiac fibrosis (Legchenko et al., 2018), establishing vascular homeostasis in PAH (Calvier et al., 2017); 6) Suppression of ERK1/2 signaling (Hansmann et al., 2008) inhibits VSMC proliferation; 7) Upregulation of the APOE-LRP8 cascade suppresses pulmonary vascular remodeling (Bertero et al., 2015).

The inventors' extended multiple-cord LC-MS analysis of prostaglandins and AA confirmed significant upregulation of PGE₂ (cells and CM) originally identified in the treated case (Figure 7D). The upregulation of PGE₂ was also supported by the integrated scRNA-seq data (upregulation of *PTGES2* and *PTGES3* in HUCMSCs) and cell proteomics data, where PTGES was below detection limit in all HUVEC samples, but was present in all HUCMSC samples.

The inventors' multiple-cord omics results raise our confidence in the validity of the componential findings, and confirm that the way we prepare MSC and conditioned media is consistent among the different batches. The latter represents an important point with respect to standardization and reproducibility.

PGE₂ signaling stimulates stem cells to regenerate damaged tissue (North et al., 2007), augments mitochondrial function and autophagy, and decreases *IFN-γ* and *TGFβ* pathways (Palla et al., 2021), which are augmented in PAH (Trembath et al., 2001; Calvier et al., 2017; Humbert et al., 2019; Morell et al., 2019). Of note, these findings have recently funneled into the development of small molecule 15-prostaglandin dehydrogenase (15-PGDH) inhibitors (SW033291) blocking PGE₂ degradation (Zhang et al., 2015), however, clinical trials are pending.

Based on both the very high PGE₂ levels found in the HUCMSC-CM, and the induction of three PGE₂ synthesis enzymes in HUCMSC, PGE₂ is considered a major beneficial component of HUCMSC-CM, with vasodilatory and regenerative properties in PAH. This is in accordance with the inventors' earlier results that concentration of high molecular weight components of the conditioned media is not advantageous, i.e., low molecular weight components such as PGE₂ are likely to be decisive.

The inventors identified a *de novo* missense mutation in the *ACVRL1* gene in the patient. Intriguingly, this particular *ACVRL1* loss-of-function-mutation (c.1451G>A, p.(Arg484Gln)) has not been found in any patients with HHT in the absence of PAH, underlining the impact of this single nucleotide variant on pulmonary vascular development and homeostasis.

The inventors' main intent was to report on the impressive improvements in the treated child and the likely mechanisms unraveled by our multi-omics analysis of the case and the available umbilical cord samples. They demonstrate safety and efficacy of MSC-derived therapy in a human patient.

In conclusion, infusions of HUCMSC-CM can lead to marked clinical and hemodynamic improvement in young patients with severe PAH. HUCMSC-derived therapy has the potential to become an efficient treatment for the most severe forms of clinical PAH.

### METHODS

### Isolation of HUCMSC and preparation of cell-derived conditioned media

### Isolation, culture, and characterization of primary HUCMSC and HUVEC.

The use of primary human MSCs following explant culture from umbilical cord tissue has been approved by the Ethics Committee of Hannover Medical School. The caregivers (parents) of the treated patient gave written informed consent for compassionate use of therapy; bioanalysis, and publication of the data. This report is in line with the CARE guidelines.

MSC-like cells were isolated from the human umbilical cord (Wharton's jelly) following delivery of a full-term (38 weeks gestation) infant (here: younger sister, by cesarian section; Figures 5 and 6), and from additional human umbilical cords (Figure 7). The cells were cultivated by explant culture in MSC growth medium (Otte et al., 2013). Briefly, umbilical cord tissue was washed several times with phosphate buffered saline (PBS) to remove blood cells, cut into approx. 1.5cm³ large pieces and incubated in MSC growth medium (αMEM (Invitrogen GmbH) supplemented with 15% of allogeneic human AB-serum (HS), 100 units/mL penicillin, 100 mg/mL streptomycin, and 2mM L-glutamine at 37°C in a humidified atmosphere with 5% CO₂). The explant culture was performed for 15 days. The outgrowth of an adherent enriched MSC population was harvested by accutase (Capricorn Scientific GmbH, Ebsdorfergrund, Germany) treatment according to the manufacturer's protocol for 5 min at 37°C. The cells were centrifuged at 320 g for 5 min, resuspended in MSC culture medium (αMEM) supplemented with 10% of HS, 100 units/mL penicillin, 100 mg/mL streptomycin, and 2mM L-glutamine at 37°C in a humidified atmosphere with 5% CO₂), and cultured at a density of 4000 cells/cm². Harvesting and subculture into corresponding passages was performed following treatment with accutase (Capricorn Scientific GmbH) at 37°C for 3 min.

Continuously proliferating MSCs were harvested and analyzed for cell cycle progression and cell surface marker expression by flow cytometry (Otte et al., 2013). Besides detectable G1, S, and G2/M phases, the presence of CD73, CD90, and CD105 with concomitant absence of CD14, CD31, CD34, CD45, and HLA-DR was tested by FACS analysis according to the suggestion by the International Society for Cellular Therapy as one of the minimal criteria for MSC characterization (Dominici et al., 2006).

Human umbilical vein endothelial cells were purchased from PromoCell (Cat#C-12200, Heidelberg, Germany), subcultured until P3 or P6 (scRNA-seq), according to manufacturer's instructions (PromoCell Instruction Manual, Heidelberg, Germany) and further served as "non-efficient" treatment control. For details, see figures and figure legends.

### Preparation of cell-derived conditioned media (CM) from HUCMSC and HUVEC

Following MSC culture in passage 2 and 3 in sub-confluent growth phase, washing out of serum with DPBS-CTS^{™} (Gibco GmbH), and addition of CTS STEMPRO MSC SFM XENOFREE basal Medium (Life Technologies, ThermoFisher GmbH), serum-free supernatant was harvested as conditioned medium (CM) after 36h, centrifuged (3.185g/10min), negatively tested for bacterial and mycoplasma contamination, and cryo-preserved at -80 °C. The night before injection (about 12h to 14h) the HUCMSC-CM was gently thawed at 4 °C and then pre-warmed to room temperature. For the HUCMSC-CM infusions, certain filters were used: Time 0, CATH #1 (i.p.a): dose 1, 200ml CM, Sterifix 0.2µm filter (B. Braun, ref. # 4099303); Time 1, CATH #2 dose 2, i.p.a.: 200mL CM, transfusion filter 200µm (B. Braun, ref. # 8270066SP). Time 1, dose 3, SVC; 200mL CM, transfusion filter 200µm (B. Braun, ref. # 8270066SP). Time 1, dose 4, SVC: 200mL CM, Minisart 0.2µm filter (Sartorius/Th. Geyer, ref # 90491011), transfusion filter 200µm (B. Braun). Time 1, dose 5, SVC: 200mL CM, Minisart 0.2µm filter (Sartorius/Th. Geyer, ref. # 90491011), transfusion filter 200µm (B. Braun). HUCMSC-CM doses 2 to 5 were applied at four consecutive days, respectively.

### Cardiac catheterizations and infusions of HUCMSC-conditioned media

After diagnosis (CATH #0), all subsequent cardiac catheterizations were performed at Hannover Medical School, i.e., in December 2019 (Time 0, CATH #1), February 2020 (Time 1, Cath #2) and May 2020 (Time 2, CATH #3; **Figure 5A****).** The PAH-targeted medication was not changed in the 6 months prior to CATH #1, when for the first time, HUCMSC-CM was administered in the pulmonary arteries, and not changed thereafter.

During CATH #1 and CATH #2, after full invasive hemodynamic assessment, 200ml HUCMSC-CM was infused in the pulmonary arteries (i.p.a.) over of 1 hour, i.e. 100 ml into the right pulmonary artery (RPA) over 30 minutes, and 100 ml into the left pulmonary artery (LPA) over 30 minutes. In the week after CATH #2, 200ml HUCMSC-CM was infused via a central venous line over 60 minutes, on days 1, 2 and 3 post CATH #2 **(****Figure 5A****).**

### Microbiological and immunological testing of HUCMSC, HUCMSC-CM, the recipient (patient), donor (sister), and mother.

*HUCMSC*/*HUCMSC-CM (cell culture supernatant)*
culture 10-14 days negative for bacterial growth (aerobic, anaerobic)

### Donor:

### HLA types:

DNA types HLA-1: A*30, A*68, B*13, B*18, C*06, C*12
DNA types HLA-2: DRB3*pos., DQB1*03
HBs-antigen negative, anti-HBC negative, anti-HBS 28IU/L
Anti-HCV negative, CMV-IgM negative, CMV-IgG 606U/ml
EBV-IgG 571 E/ml, VCA IgM negative, EBNA1-IgG 141 E/ml
Toxoplasma screening test negative
Treponema pallidum IA-test negative
HIV-AK1/2, p24-Ag negative

### Recipient:

Blood type 0 Rh positive, CcDD.Ee, K-, irregular RBC antibody negative

### HLA types:

DNA types HLA-1: A*24, A*68, B*13, B*18, C*06, C*12
DNA types HLA-2: DRB1*07, DRB1*11, DRB3*pos., DRB4*pos., DQB1*02, DQB1*03
GvH constellation (MSC transplant vs. patient): A*24, C*04, DRB1*07, DQB1*02
HvG constellation (patient against MSC transplant): A*68, C*06
Mycoplasma IgM negative, mycoplasma IgG negative

### Single-cell sequencing of MSC and HUVEC cell samples

Library preparation for single cell mRNA-Seq analysis was performed according to the Chromium NextGEM Single Cell 3' Reagent Kits v3.1 User Guide (Manual Part Number CG000204 Rev B; 10x Genomics). A twofold excess of cells was loaded to the 10x controller in the specified volume in order to reach a target number of 1500 cells per sample. Equal molar proportions of eight generated libraries were pooled accordingly, denatured with NaOH, and were finally diluted to 1.8pM according to the 'Denature and Dilute Libraries Guide' (Document # 15048776 v02; Illumina). 1.3 ml of denatured pool was sequenced on an Illumina NextSeq 550 sequencer using one High Output Flowcell for 75 cycles and 400 Million clusters (#20024906; Illumina). The proprietary 10x Genomics CellRanger pipeline (v4.0.0) was used with default parameters except for the setting of expected cells (--expect-cells 1500). CellRanger was used to align read data to the human reference genome provided by 10XGenomics (refdata-gex-GRCh38-2020-A) using the STAR aligner. Mean number of reads per cell ranged from 29916 to 42827 across all samples. Median number of genes per cell ranged from 3578 to 4465 across all samples.

**Enzyme-Linked Immunosorbent Assays (ELISA)** The blood samples were immediately centrifuged for 10 min at 1300g. Plasma was aliquoted and stored at -80 °C. Plasma samples were diluted 1:3 with Sample Diluent and plasma neural precursor cell expressed developmentally down-regulated protein 9 (NEDD9) concentrations were determined according to the manufacturer's instructions (Aviva Systems Biology, San Diego, CA, OKEH02459, Lot KE0777). Briefly, 100 uL of standards, diluted samples and blank were added into the wells of the NEDD9 microplate and incubated at 37° for 2 hours. Liquid was discarded and 100uL of biotinylated NEDD9 Detector Antibody was added to each well and incubated at 37°C for 60 minutes. Liquid was removed and the microplate washed with Wash Buffer. 100uL of Avidin-HRP Conjugate was added to each well and incubated at 37°C for 60 minutes, followed by another washing step. 50ul TMB Substrate was added and incubated at 37°C in the dark for 15 minutes. Finally, 50 uL of Stop Solution was added to each well and the absorbance was read at 450 nm with a wavelength correction of 570 nm.

Plasma ICAM-1 (sample dilution 1:1000), SAA (sample dilution 1:1000), IFN-γ (sample dilution 1:2) concentrations were measured by applying Meso Scale Discovery's Multi-Array technology, according to the manufacturer's instructions. ICAM-1 and SAA were measured within Vascular Injury Panel (K15198D-1) and IFN-γ was detected within Proinflammatory Panel (K15049D-1), both according to manufacturer's instructions. Briefly, the plates were washed thrice with 150ul/well wash buffer, and 25uL of diluted sample, calibrator or control were added per well (for ICAM-1 and SAA) or 50ul of diluted sample, calibrator or control for IFN-γ. The plates then were incubated at room temperature for 2 hours with shaking. After incubation plates wer washed 3 times with 150ul/well wash buffer, 25ul of detection antibody was added to each well, and the plate was incubated for additional 1 hour (for ICAM-1 and SAA) or 2 hours for IFN-γ with shaking at room temperature. Finally, the plates were washed 3 times with 150ul/well wash buffer, 150ul of 1X Read Buffer was added per well (for ICAM-1 and SAA) or 2X Read Buffer for IFN-γ. Signal intensities were detected and analyzed with a MESO QuickPlex SQ 120 instrument and Discovery Workbench software V.4.0 (MSD, Rockville, Maryland, USA). The average protein concentrations from the superior (SVC) and inferior (IVC) vena cava and the right atrium (RA) are reported.

### Label-free quantitative discovery proteomics

### Sample preparation for proteomics

Protein was extracted from HUVEC and HUCMSC cells and DNA sheared in 40 µl lysis buffer (1% SDS, 0.1 M ABC, 1,25x PIC) in AFA-TUBE TPX Strips on a Covaris LE220Rsc by focused ultrasonication (PIP 450 W, DF 25%, CPB 200, 2 repeats, 300 s pulse, 20 C). Samples were cleared from debris (2500 × g for 5 min) and protein quantified (Pierce BCA, 23225). Samples of 30 µg cellular protein were filled to 50 µl with lysis buffer and 16.6 µl reduction and alkylation buffer (40 mM TCEP, 160 mM CAA, 200mM ABC) were added. Secreted proteins in the conditioned media (200 µl) were concentrated (overnight lyophilisation) and reconstituted in 40 µl 10 mM TCEP, 40 mM CAA. Cellular and secreted proteins were prepared using the SP3 protocol with single-step reduction and alkylation (Muller et al., 2020) on a Beckmann Biomek i7 workstation. Samples were incubated for 5 min at 95 °C and cooled to RT. Proteins were bound to 250 µg paramagnetic beads (1:1 ratio of of hydrophilic/hydrophobic beads) by adding acetonitrile (ACN) to 50% for cellular or 70% for secreted proteins respectively. Samples were washed twice with 80% ethanol and once with 100% ACN, before reconstitution in 35 µl 100 mM ABC. Digestion was completed overnight at 37°C using a trypsin/ LysC enzyme mix (Promega, Madison, WI, USA) at a ratio of protein:enzyme of 50:1 for cellular and 250 ng for secreted proteins respectively. The reaction was stopped with formic acid (0.1%) and the peptides stored at -80°C until analysis without further conditioning or clean-up.

### Proteome analysis by DIA LC-MS

The amount of injected tryptic digest was set to 40 ng, the available material for the lowest concentrated sample. Peptides were resolved on a 25 cm Aurora Series with emitter column (CSI, 25cm × 75µm ID, 1.6pm C18, IonOpticks, installed in the nano-electrospray source (CaptiveSpray source, Bruker Daltonics, Germany) at 50°C using an UltiMate 3000 (Thermo Scientific Dionex) coupled with TIMS quadrupole time-of-flight instrument (timsTOF Pro2, Bruker Daltonics, Germany) and measured in diaPASEF mode. The mobile phases Water/0.1% FA and ACN/0.1% FA (A and B respectively) were applied in the linear gradients starting from 2% B and increasing to 17% in 87 min, followed by an increase to 25% B in 93min, 37% B in 98 min, 80% B in 99 min to 104 min, the column was equilibrated in 2% B by next 15 min. For calibration of ion mobility dimension, three ions of Agilent ESI-Low Tuning Mix ions were selected (m/z [Th], 1/*K*0 [Th]: 622.0289, 0.9848; 922.0097, 1.1895; 1221.9906, 1.3820). The diaPASEF windows scheme was ranging in dimension m/z from 396 to 1103 Th and in dimension 1/*K*0 0.7 - 1.3 Vs cm- 2, with 59 × 12 Th windows). All measurements were done in Low Sample Amount Mode with Ramp Time 166 ms.

### Protein identification and quantification

The raw data was processed using DIA-NN 1.8 (Demichev et al., 2020) with the ion mobility module for diaPASEF (Demichev et al 2021 bioRxiv, 10.1101/2021.03.08.434385). MS2 and MS1 mass accuracies were both set to 10 ppm, and scan window size to 10. DIA-NN was run in library-free mode with standard settings (fasta digest and deep learning-based spectra, RT and IMs prediction) using the uniprot human reference proteome annotations (UP000005640_9606, downloaded on 2019-12-20) (UniProt, 2019) und the match-between-runs (MBR) option.

### Liquid chromatography - mass spectrometry of cells and conditioned media

Samples were spiked with internal standards to a final concentration of 1.0 ng/mL and prepared using solid phase extraction as described elsewhere (Jensen et al., 2022). 200 µL conditioned medium samples were used. Medium samples were reconstituted in 200 µL 40% MeOH and cell pellet samples in 100 µL 40% MeOH. LC-MS analysis was carried out using two LC-30AD pumps, a SIL-30AC autosampler and a CTO-20AC column oven (all Shimadzu, 's Hertogenbosch, The Netherlands). The autosampler was held at 6 °C. Forty µL was injected and separation was accomplished on a Kinetex C18 column (Phenomenex, Aschaffenburg, Germany, 50 × 2.1 mm, 1.7 µm) using a gradient of 0.01% acetic acid (Fluka, Darmstadt, Germany) in water (Honeywell-Riedel de Haën, Seelze, Germany; A) and 0.01% acetic acid in MeOH (B). The oven was held at 50 °C. The gradient was as follows: 0.0 - 1.0 min. constant at 30% B, 1.0 - 1.1 min. linear increase to 45% B, 1.1 - 2.0 min. linear increase to 53.5% B, 2.0 - 4.4 min. linear increase to 55.5% B, 4.0 - 7.0 min. linear increase to 90% B, 7.0 - 7.1 min. linear increase to 100% B, 7.1 - 9.0 min. constant at 100% B, 9.0 - 9.5 min. linear decrease to 30% B, 9.5 - 11.5 min. constant at 30% B. Detection was achieved on a Qtrap 6500 (Sciex Nieuwerkerk a/d IJssel, The Netherlands) equipped with a ESI source. The MS was operated in negative scheduled MRM mode. The needle voltage of the source was set at - 4500 V, the drying temperature at 450 °C, ion source gas 1 and 2 (both air) at respectively 40 and 30 psi and the nebulizer gas (nitrogen) at 30 psi. The entrance potential was set to 10 V and the collision gas flow to 'medium'. Detailed settings can be found elsewhere (Gart et al., 2021). Calibration ranges and functions are given in Supplementary Tables 6 and 7. All calibration lines were weighed 1/x². Results were expressed as ng/mL or ng prostaglandin per mg protein (cell pellet). Protein was quantified using the BCA assay according to the manufacturer's instructions.

To confirm the regenerative, MSC-derived impact of PGE₂, the inventors only included a small panel of selected prostaglandins (PGD₂, PGF_{2α}, PGE₂, 8-iso-PGE₂, 8-iso-PGF_{2α}) and their precursor arachidonic acid (AA) in the LC-MS/MS analysis.

### Statistical analysis

Single-cell RNA-seq (scRNA-seq) data analysis was performed using the Seurat R package (v. 4.0.2). Two types of scRNA-seq analysis were performed: 1) The HUCMSC cells that generated the conditioned media used for treating the reported case (sample ID: HUCMSC1_P3_female; cell number: 1418) were analyzed for presence of cell clusters with regeneration potential. Following the standard analysis steps in Seurat (as outlined in the manual), including regressing out the cell cycle effects, we performed unsupervised clustering of the single cell data. 2) Integrated scRNA-seq analysis of three other HUCMSC cell samples (sample IDs: HUCMSC2_P6_male, HUCMSC3_P6_male, HUCMSC4_P6_male; respective cell numbers: 1317, 1982, 3203) and two HUVEC cell samples (sample IDs: HUVEC1_P6_male, HUVEC2_P6_male; respective cell numbers: 1316, 2397) was performed as outlined in the Seurat tutorial (https://satijalab.org/seurat/archive/v3.1/immune alignment.html). Prior to this analysis, standard filtration was performed. However, given that the difference in proliferation rates between HUCMSCs and HUVECs may be biologically relevant, we chose not to regress out the cell cycle effects. Batch effect correction and normalization was performed using the SCTransform function from the SCTransform R package (ver. 0.3.3). Percentage of mitochondrial genes and sample IDs were used as variables to regress out in SCTransform. Upon batch effect correction, samples percentages per cluster ranged as follows: Cluster 0 (16.87 - 21.67%), Cluster 1 (18.47 - 20.9%), Cluster 2 (15.02 - 25.17%), Cluster 3 (18.53 - 21.01%), Cluster 4 (13.19 -26.44%), Cluster 5 (16.14 -23.87%), Cluster 6 (13.03 -27.58%), Cluster 7 (0 -40.44%). Seurat's function SelectlntegrationFeatures with the number of features set to 3000 was used for feature selection. Seurat's ElbowPlot function was used to estimate the number of meaningful dimensions. The marker genes used for definition of the cell clusters or differentially expressed genes (false discovery rate adjusted p-values < 0.05) from the integrated analysis (HUCMSC vs. HUVEC) were analyzed for GO and pathway overrepresentation using the online tool Enrichr (https://maayanlab.cloud/Enrichr/). Differentially expressed genes were identified using the FindMarkers function with default parameters from the Seurat package.

Proteomics analysis was performed using the DEP R package (v. 1.12.0) with default parameters. Further, we used GSEA (v. 4.1.0) with default parameters to perform gene enrichment set analysis of differentially expressed genes and differentially enriched proteins. Prostaglandin (LC-MS) results were analyzed using GraphPad Prism (v. 7). Two-tailed Mann Whitney U tests were used, since normality could not be checked due to small sample sizes.

### Data availability

The scRNA-seq data are accessible via NCBI Gene Expression Omnibus (accession ID: GSE199071). We have deposited the raw data for proteomics experiments to PRIDE (EMBL), which is a part of ProteomeXchange (accession ID: PXD032234).

### References

Galie, N. et al. Risk stratification and medical therapy of pulmonary arterial hypertension. Eur Respir J 53, doi:10.1183/13993003.01889-2018 (2019).
Hansmann, G. et al. 2019 updated consensus statement on the diagnosis and treatment of pediatric pulmonary hypertension: The European Pediatric Pulmonary Vascular Disease Network (EPPVDN), endorsed by AEPC, ESPR and ISHLT. J Heart Lung Transplant 38, 879-901, doi:10.1016/j.healun.2019.06.022 (2019).
Hansmann, G. Pulmonary hypertension in infants, children, and young adults. J Am Coll Cardiol 69, 2551-2569, doi:10.1016/j.jacc.2017.03.575 (2017).
Trembath, R. C. et al. Clinical and molecular genetic features of pulmonary hypertension in patients with hereditary hemorrhagic telangiectasia. The New England journal of medicine 345, 325-334, doi:10.1056/NEJM200108023450503 (2001).
Calvier, L. et al. PPARgamma links BMP2 and TGFbeta1 pathways in vascular smooth muscle cells, regulating cell proliferation and glucose metabolism. Cell Metab 25, 1118-1134 e1117, doi:10.1016/j.cmet.2017.03.011 (2017).
Humbert, M. et al. Pathology and pathobiology of pulmonary hypertension: state of the art and research perspectives. Eur Respir J 53, doi:10.1183/13993003.01887-2018 (2019).
Morrell, N. W. et al. Genetics and genomics of pulmonary arterial hypertension. Eur Respir J 53, doi:10.1183/13993003.01899-2018 (2019).
Girerd, B. et al. Clinical outcomes of pulmonary arterial hypertension in patients carrying an ACVRL1 (ALK1) mutation. Am J Resp Crit Care Med 181, 851-861, doi:10.1164/rccm.200908-1284OC (2010). Legchenko, E. etal. The PPARgamma agonist pioglitazone reverses pulmonary hypertension and prevents right heart failure via fatty acid oxidation. Sci Transl Med 10, eaao0303 (2018).
Zelt JGE et al. Medical Therapy for Heart Failure Associated With Pulmonary Hypertension. Circ Res 2019;124(11):1551-1567 (2019).
Granton, J. et al. Endothelial NO-Synthase Gene-Enhanced Progenitor Cell Therapy for Pulmonary Arterial Hypertension: The PHACeT Trial. Circ Res 117, 645-654, doi:10.1161/CIRCRESAHA.114.305951 (2015). Klinke, A. et al. Emerging therapies for right ventricular dysfunction and failure. Cardiovasc Diagn Ther 10, 1735-1767, doi:10.21037/cdt-20-592 (2020).
Hansmann G et al. Mesenchymal stem cell-mediated reversal of bronchopulmonary dysplasia and associated pulmonary hypertension. Pulm Circ 2012;2(2):170-81 (2012).
Shah, R. et al. Circulating Extracellular Vesicles in Human Disease. N Engl J Med 379, 2180-2181, doi:10.1056/NEJMc1813170 (2018).
Willis, G. R. et al. Mesenchymal Stromal Cell Exosomes Ameliorate Experimental Bronchopulmonary Dysplasia and Restore Lung Function through Macrophage Immunomodulation. Am J Respir Crit Care Med 197, 104-116, doi:10.1164/rccm.201705-0925OC (2018).
Klinger, J. R. et al. Mesenchymal Stem Cell Extracellular Vesicles Reverse Sugen/Hypoxia Pulmonary Hypertension in Rats. Am J Respir Cell Mol Biol 62, 577-587, doi:10.1165/rcmb.2019-0154OC (2020). Mitsialis, S. A. The Unsettling Ambiguity of Therapeutic Extracellular Vesicles from Mesenchymal Stromal Cells. Am J Respir Cell Mol Biol 62, 539-540, doi:10.1165/rcmb.2019-0382ED (2020).
Simonneau G et al. Haemodynamic definitions and updated clinical classification of pulmonary hypertension. Eur Respir J 2019;53(1).
Galie N et al. Group ESCSD. 2015 ESC/ERS Guidelines for the diagnosis and treatment of pulmonary hypertension: The Joint Task Force for the Diagnosis and Treatment of Pulmonary Hypertension of the European Society of Cardiology (ESC) and the European Respiratory Society (ERS): Endorsed by: Association for European Paediatric and Congenital Cardiology (AEPC), International Society for Heart and Lung Transplantation (ISHLT). Eur Heart J 2016;37(1):67-119 (2016)
Hass, R et al. Different populations and sources of human mesenchymal stem cells (MSC): A comparison of adult and neonatal tissue-derived MSC. Cell Commun Signal 9, 12, doi:10.1186/1478-811X-9-12 (2011). Yang Y et al. Conditioned umbilical cord tissue provides a natural three-dimensional storage compartment as in vitro stem cell niche for human mesenchymal stroma/stem cells. Stem Cell Res Ther 2016;7:28 (2016).
Hoffmann A et al. Comparison of in vitro-cultivation of human mesenchymal stroma/stem cells derived from bone marrow and umbilical cord. J Tissue Eng Regen Med 2017;11(9):2565-2581 (2017)
Melzer C et al. Anti-Tumor Effects of Exosomes Derived from Drug-Incubated Permanently Growing Human MSC. Int J Mol Sci 2020;21(19) (2020).
Dominici, M. et al. Minimal criteria for defining multipotent mesenchymal stromal cells. The International Society for Cellular Therapy position statement. Cytotherapy 8, 315-317 (2006).
Zhang Y et al. TISSUE REGENERATION. Inhibition of the prostaglandin-degrading enzyme 15-PGDH potentiates tissue regeneration. Science 2015;348(6240):aaa2340 (2015).
Shovlin, C. L. et al. Diagnostic criteria for hereditary hemorrhagic telangiectasia (Rendu-Osler-Weber syndrome). Am J Med Genet 91, 66-67, doi:10.1002/(sici)1096-8628(20000306)91:1<66::aid-ajmg12>3.0.co;2-p (2000).
Faughnan, M. E. et al. International guidelines for the diagnosis and management of hereditary haemorrhagic telangiectasia. J Med Genet 48, 73-87, doi:10.1136/jmg.2009.069013 (2011).
Lavrentieva etal. Effects of hypoxic culture conditions on umbilical cord-derived human mesenchymal stem cells. Cell Commun Signal 8, 18, doi:10.1186/1478-811X-8-18 (2010).
Samokhin, A. O. et al. NEDD9 targets COL3A1 to promote endothelial fibrosis and pulmonary arterial hypertension. Sci Transl Med 10, doi:10.1126/scitranslmed.aap7294 (2018).
Alba, G. A. et al. NEDD9 Is a Novel and Modifiable Mediator of Platelet-Endothelial Adhesion in the Pulmonary Circulation. Am J Respir Crit Care Med 203, 1533-1545, doi:10.1164/rccm.202003-0719OC (2021).
Samokhin, A. O. et al. Circulating NEDD9 is increased in pulmonary arterial hypertension: A multicenter, retrospective analysis. J Heart Lung Transplant 39, 289-299, doi:10.1016/j.healun.2019.12.002 (2020). Calvier, L. et al. Galectin-3 and aldosterone as potential tandem biomarkers in pulmonary arterial hypertension. Heart 102, 390-396 (2016).
Akhmetshina, A. et al. Activation of canonical Wnt signalling is required for TGF-beta-mediated fibrosis. Nat Commun 3, 735, doi:10.1038/ncomms1734 (2012).
Tsaousi, A. et al. Wnt4/beta-catenin signaling induces VSMC proliferation and is associated with intimal thickening. Circ Res 108, 427-436, doi:10.1161/CIRCRESAHA.110.233999 (2011).
Nakamura, M. et al. Glycogen Synthase Kinase-3alpha Promotes Fatty Acid Uptake and Lipotoxic Cardiomyopathy. Cell Metab 29, 1119-1134 e1112, doi:10.1016/j.cmet.2019.01.005 (2019).
Hansmann, G. et al. An antiproliferative BMP-2/PPARgamma/apoE axis in human and murine SMCs and its role in pulmonary hypertension. J Clin Invest 118, 1846-1857 (2008).
Yu, K. R. et al. A p38 MAPK-mediated alteration of COX-2/PGE2 regulates immunomodulatory properties in human mesenchymal stem cell aging. PLoS One 9, e102426, doi:10.1371/journal.pone.0102426 (2014). North, T. E. et al. Prostaglandin E2 regulates vertebrate haematopoietic stem cell homeostasis. Nature 447, 1007-1011, doi:10.1038/nature05883 (2007).
Hansmann, G. et al. Pulmonary arterial hypertension is linked to insulin resistance and reversed by peroxisome proliferator-activated receptor-gamma activation. Circulation 115, 1275-1284 (2007).
Calvier, L., Boucher, P., Herz, J. & Hansmann, G. LRP1 Deficiency in Vascular SMC Leads To Pulmonary Arterial Hypertension That Is Reversed By PPARgamma Activation. Circ Res, doi:10.1161/CIRCRESAHA.119.315088 (2019).
Fujiu, K. et al. Fibroblast-mediated pathways in cardiac hypertrophy. J Mol Cell Cardiol 70, 64-73, doi:10.1016/j.yjmcc.2014.01.013 (2014).
Bertero, T. et al. Matrix Remodeling Promotes Pulmonary Hypertension through Feedback Mechanoactivation of the YAP/TAZ-miR-130/301 Circuit. Cell Rep 13, 1016-1032, doi:10.1016/j.celrep.2015.09.049 (2015).
Palla, A. R. et al. Inhibition of prostaglandin-degrading enzyme 15-PGDH rejuvenates aged muscle mass and strength. Science 371, doi:10.1126/science.abc8059 (2021).
Otte, A. et al. Mesenchymal stem cells maintain long-term in vitro stemness during explant culture. Tissue Eng Part C Methods 19, 937-948, doi:10.1089/ten.TEC.2013.0007 (2013).
Muller, T. et al. Automated sample preparation with SP3 for low-input clinical proteomics. Mol Syst Biol 16, e9111, doi:10.15252/msb.20199111 (2020).
Demichev, V et al. DIA-NN: neural networks and interference correction enable deep proteome coverage in high throughput. Nat Methods 17, 41-44, doi:10.1038/s41592-019-0638-x (2020).
UniProt, C. UniProt: a worldwide hub of protein knowledge. Nucleic Acids Res 47, D506-D515, doi:10.1093/nar/gky1049 (2019).
Jensen, K. N. et al. Dietary Fish Oil Increases the Number of CD11b(+)CD27(-) NK Cells at the Inflammatory Site and Enhances Key Hallmarks of Resolution of Murine Antigen-Induced Peritonitis. J Inflamm Res 15, 311-324, doi:10.2147/JIR.S342399 (2022).
Gart, E. et al. Krill Oil Treatment Increases Distinct PUFAs and Oxylipins in Adipose Tissue and Liver and Attenuates Obesity-Associated Inflammation via Direct and Indirect Mechanisms. Nutrients 13, doi:10.3390/nu13082836 (2021).
Nickel et al. AJRCCM; 2020; Beyond the Lungs: Systemic Manifestations of Pulmonary Arterial Hypertension, doi: 10.1164/rccm.201903-0656Cl (2020)
Hamberger F et al. Front Cardiovasc Med. 2022 Mar 17;9:862330. doi: 10.3389/fcvm.2022.862330. eCollection 2022. PMID: 35369312 (2022)
Rosenkranz et al. 2020; Circulation. 2020;141:678-693. doi: 10.1161/CIRCULATIONAHA.116.022362 (2020)
Zamanian RT etal. Insulin resistance in pulmonary arterial hypertension. Eur Respir J. 2009 Feb;33(2):318-24. doi: 10.1183/09031936.00000508. Epub 2008 Dec 1. PMID: 19047320 (2009)
Poms etal. Comorbid conditions and outcomes in patients with pulmonary arterial hypertension: a REVEAL registry analysis. Chest 2013;144:169-176 (2013).
Agrawal V et al. Molecular mechanisms of right ventricular dysfunction in pulmonary arterial hypertension: focus on the coronary vasculature, sex hormones, and glucose/lipid metabolism. Cardiovasc Diagn Ther. 2020 Oct;10(5):1522-1540. doi: 10.21037/cdt-20-404. PMID: 33224772 Free PMC article. Review (2020). Hansmann G et al. Nature Cardiovascular Research 2022; in press, to be published on June 9, 2022; doi: 10.1038/s44161-022-00083-z (2022).

## Claims

1. A human mesenchymal stem cell-conditioned medium for use in treatment of a chronic heart-lung and vascular disease in a human subject.

2. The mesenchymal stem cell-conditioned medium of claim 1, wherein the chronic heart-lung and vascular disease is selected from the group consisting of pulmonary hypertension, pulmonary fibrosis, chronic obstructive pulmonary disease, bronchopulmonary dysplasia, and other chronic progressive lung diseases and chronic heart and vascular disease.

3. The mesenchymal stem cell-conditioned medium of claim 2, wherein the chronic heart-lung disease is pulmonary hypertension.

4. The mesenchymal stem cell-conditioned medium of claim 3, wherein the pulmonary hypertension is pulmonary arterial hypertension, optionally, severe and progressive pulmonary arterial hypertension.

5. The mesenchymal stem cell-conditioned medium of claim 3, wherein the pulmonary hypertension is severe and progressive pulmonary arterial hypertension selected from the group consisting of pulmonary arterial hypertension associated with at least one mutation in a pulmonary arterial hypertension candidate gene, a proinflammatory condition such as systemic sclerosis, and pulmonary arterial hypertension in a young patient.

6. The mesenchymal stem cell-conditioned medium of claim 5, wherein pulmonary arterial hypertension is associated with at least one mutation in a pulmonary arterial hypertension candidate gene selected from the group consisting of *ACVRL1, BMPR2, ENG, CAV1, EDN1, SMAD4, SMAD9, AGTR1, BMPR1B, EDNRA, EIF2AK4, KCNA5, KCNK3, NOS2, NOTCH3, SERPINE1, SIRT3, SOX17, TBX4, THBS1, TOPBP1* and TRPC6 genes., preferably, associated with a mutation in the *ACVRL1* gene.

7. The mesenchymal stem cell-conditioned medium of any of the preceeding claims, wherein the mesenchymal stem cells are umbilical cord mesenchymal stem cells.

8. The mesenchymal stem cell-conditioned medium of any of the preceeding claims, wherein the conditioned medium comprises PGE₂, preferably, in an amount of at least 100 ng PGE₂/mg protein, wherein the conditioned medium optionally further comprises PGF_{2α}, preferably, in an amount of at most 18 ng PGF_{2α}/mg protein.

9. The mesenchymal stem cell-conditioned medium of any of the preceeding claims, wherein the conditioned medium comprisesLRP1, APOE, MAPK11, FGF16, and LRP8.

10. The mesenchymal stem cell-conditioned medium of any of the preceeding claims, wherein the conditioned medium comprises extracellular vesicles, wherein, preferably, it does not essentially consist of extracellular vesicles.

11. The mesenchymal stem cell-conditioned medium of any of the preceeding claims, wherein the conditioned medium has not been concentratred via filtration.

12. The mesenchymal stem cell-conditioned medium of any of the preceeding claims, wherein the conditioned medium is serum-free medium harvested from a sub-confluent culture of mesenchymal stem cells after 12 to 60 hours of culture, optionally, after 24 to 48 hours of culture.

13. The mesenchymal stem cell-conditioned medium of any of the preceeding claims, wherein the treatment comprises administering the conditioned medium intravenously, to a pulmonary artery of the subject and/or intracoronaryly.

14. The mesenchymal stem cell-conditioned medium of any of the preceeding claims, wherein the treatment comprises administering a dose of 10 to 2000 mL of the conditioned medium, preferably, 100 to 500 mL.

15. The mesenchymal stem cell-conditioned medium of any of the preceeding claims, wherein the treatment comprises administering one to ten doses with an interval of at least one day between doses, optionally, three to five doses.
